# EUROPEAN PATENT SPECIFICATION

(11) **EP 4 010 329 B1**
(45) Date of publication and mention of the grant of the patent: **07.06.2023**
(21) Application number: 20754011.3
(22) Date of filing: 10.08.2020
(51) Int. Cl.: C07D 401/04, A61K 31/4439, A61P 35/00

(54) **DEUTERATED COMPOUNDS FOR USE IN THE TREATMENT OF CANCER**
DEUTERIERTE VERBINDUNGEN ZUR VERWENDUNG BEI DER BEHANDLUNG VON KREBS
COMPOSÉS DEUTÉRÉS DESTINÉS À ÊTRE UTILISÉS DANS LE TRAITEMENT DU CANCER

(30) Priority: 09.08.2019 WO PCT/GB2019/052240; 06.12.2019 GB 201917863
(43) Date of publication of application: 15.06.2022
(73) Proprietor: Artios Pharma Limited, Cambridge CB22 3AT (GB)
(72) Inventor: BLENCOWE, Peter, Cambridge CB22 3AT (GB); CHARLES, Mark, Cambridge CB22 3AT (GB); EKWURU, Tennyson, Cambridge CB22 3AT (GB); FINCH, Harry, Cambridge CB22 3AT (GB); MCCARRON, Hollie, Cambridge CB22 3AT (GB); HEALD, Robert, Cambridge CB22 3AT (GB); STOCKLEY, Martin, Cambridge CB22 3AT (GB)
(74) Representative: Gibson, Mark
(86) International application number: PCT/GB2020/051901
(87) International publication number: WO 2021/028670

(56) References cited:
- WO-A1-2013/107291
- WO-A1-2013/107405
- WO-A1-2015/010297
- WO-A1-2018/001332
- JANETA POPOVICI-MULLER ET AL: "Discovery of AG-120 (Ivosidenib): A First-in-Class Mutant IDH1 Inhibitor for the Treatment of IDH1 Mutant Cancers", ACS MEDICINAL CHEMISTRY LETTERS, vol. 9, no. 4, 12 April 2018 (2018-04-12), pages 300-305, XP055630303, US ISSN: 1948-5875, DOI: 10.1021/acsmedchemlett.7b00421

## Description

### FIELD OF THE INVENTION

The invention relates to heterocyclic amide derivatives and their use in the treatment and prophylaxis of cancer, and to compositions containing said derivatives and processes for their preparation.

### BACKGROUND OF THE INVENTION

Robust repair of DNA double-strand breaks (DSBs) is essential for the maintenance of genome stability and cell viability. DSBs can be repaired by one of three main pathways: homologous recombination (HR), non-homologous end-joining (NHEJ) and alternative NHEJ (alt-NHEJ). Microhomology-mediated end-joining (MMEJ) is the most well characterised alt-NHEJ mechanism. HR-mediated repair is a high-fidelity mechanism essential for accurate error-free repair, preventing cancer-predisposing genomic stability. Conversely, NHEJ and MMEJ are error-prone pathways that can leave mutational scars at the site of repair. MMEJ can function parallel to both HR and NHEJ pathways (Truong et al. PNAS 2013, 110 (19), 7720-7725).

The survival of cancer cells, unlike normal cells, is often dependent on the mis-regulation of DNA damage response (DDR) pathways. For example, an increased dependency on one pathway (often mutagenic) to cope with either the inactivation of another one, or the enhanced replication stress resulting from increased proliferation. An aberrant DDR can also sensitise cancer cells to specific types of DNA damage, thus, defective DDR can be exploited to develop targeted cancer therapies. Crucially, cancer cells with impairment or inactivation of HR and NHEJ become hyper-dependent on MMEJ-mediated DNA repair. Genetic, cell biological and biochemical data have identified Polθ (UniProtKB - 075417 (DPOLQ_HUMAN) as the key protein in MMEJ (Kent et al. Nature Structural & Molecular Biology (2015), 22(3), 230-237, Mateos-Gomez et al. Nature (2015), 518(7538), 254-257). Polθ is multifunctional enzyme, which comprises an N-terminal helicase domain (SF2 HEL308-type) and a C-terminal low-fidelity DNA polymerase domain (A-type) (Wood & Doublié DNA Repair (2016), 44, 22-32). Both domains have been shown to have concerted mechanistic functions in MMEJ. The helicase domain mediates the removal of RPA protein from ssDNA ends and stimulates annealing. The polymerase domain extends the ssDNA ends and fills the remaining gaps.

Therapeutic inactivation of Pοlθ would thus disable the ability of cells to perform MMEJ and provide a novel targeted strategy in an array of defined tumour contexts. Firstly, Pοlθ has been shown to be essential for the survival of HR-defective (HRD) cells (e.g. synthetic lethal with FA/BRCA-deficiency) and is up-regulated in HRD tumour cell lines (Ceccaldi et al. Nature (2015), 518(7538), 258-262). *In vivo* studies also show that Pοlθ is significantly over-expressed in subsets of HRD ovarian, uterine and breast cancers with associated poor prognosis (Higgins et al. Oncotarget (2010), 1, 175-184, Lemée et al. PNAS (2010), 107(30), 13390-13395, Ceccaldi *et al.* (2015), *supra*)*.* Importantly, Pοlθ is largely repressed in normal tissues but has been shown to be upregulated in matched cancer samples thus correlating elevated expression with disease (Kawamura et al. International Journal of Cancer (2004), 109(1), 9-16). Secondly, its suppression or inhibition confers radio-sensitivity in tumour cells. Finally, Pοlθ inhibition could conceivably prevent the MMEJ-dependent functional reversion of BRCA2 mutations that underlies the emergence of cisplatin and PARPi resistance in tumours.

There is therefore a need to provide effective Pοlθ inhibitors for the treatment of cancer.

### SUMMARY OF THE INVENTION

According to a first aspect of the invention, there is provided a deuterated derivative of a compound of formula (I): 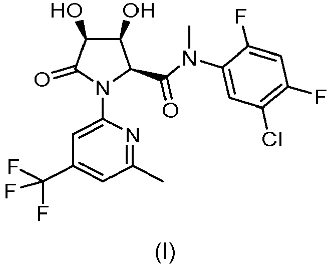 or a tautomeric or a stereochemically isomeric form thereof, or a pharmaceutically acceptable solvate thereof.

### BRIEF DESCRIPTION OF THE FIGURES

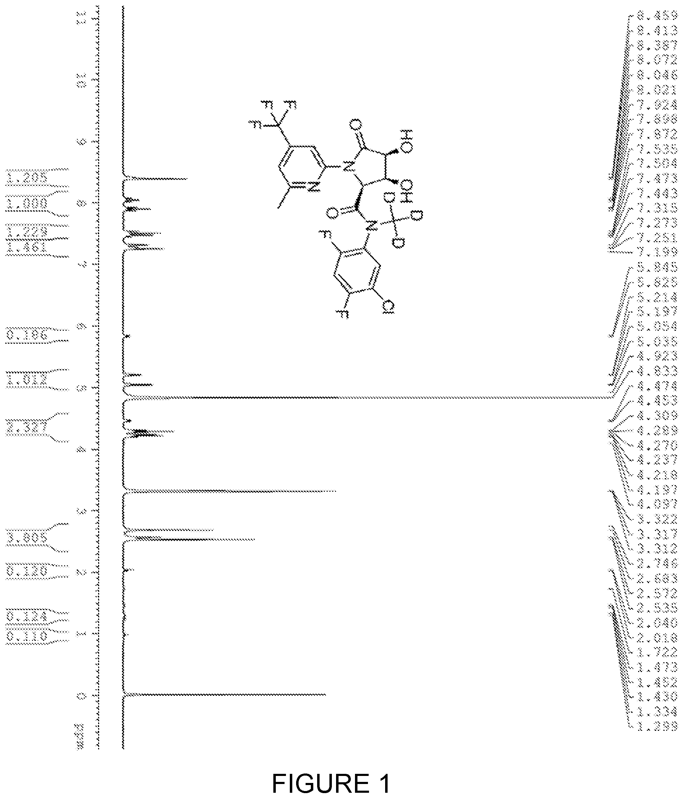

Figure 1: ¹H NMR spectra of Example 1 (300 MHz, CD₃OD, 300.6 K).

### DETAILED DESCRIPTION OF THE INVENTION

References herein to "deuterated derivative" refers to any compound of formula (I) wherein any one or more (such as 1, 2 or 3) hydrogen atoms are substituted with the heavier stable isotope deuterium, *i.e.* ²H (D).

Thus, in one embodiment, the deuterated derivative comprises deuteration of 1, 2 or 3 hydrogen atoms. In a further embodiment, the deuterated derivative comprises deuteration of 1, 2 or 3 hydrogen atoms of the N-methyl group of said compound of formula (I). In a yet further embodiment, the deuterated derivative comprises deuteration of all 3 hydrogen atoms of the N-methyl group of said compound of formula (I).

Thus, in a yet further embodiment, the deuterated derivative is a compound of formula (II): 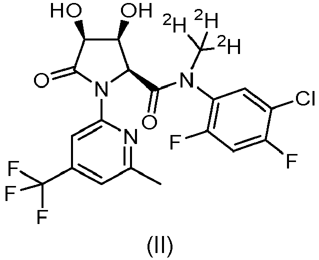 or a tautomeric or a stereochemically isomeric form, or a pharmaceutically acceptable solvate thereof.

In a still yet further embodiment, the compound of formula (II) is the free base of a compound of formula (II) and is (2S,3S,4S)-N-(5-chloro-2,4-difluorophenyl)-3,4-dihydroxy-N-(methyl-d₃)-1-(6-methyl-4-(trifluoromethyl)pyridin-2-yl)-5-oxopyrrolidine-2-carboxamide (E1). A reference to a compound of the formula (I) and sub-groups thereof also includes solvates, stereochemical isomers, tautomers, and isotopes thereof, for example, as discussed below; preferably, the tautomers or stereoisomers or solvates thereof; and more preferably, the tautomers or solvates thereof, even more preferably the tautomers or solvates thereof. Hereinafter, compounds and their solvates, stereochemical isomers, tautomers, and isotopes (such as deuterated derivatives thereof) as defined in any aspect of the invention (except intermediate compounds in chemical processes) are referred to as "compounds of the invention".

### Solvates

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Pharmaceutically acceptable solvates of the compound of the invention are within the scope of the invention. In one embodiment, the pharmaceutically acceptable solvates of the compounds of the invention include the hydrate thereof.

In one embodiment, said crystalline form of the compounds of formula (I) is a cocrystal or coformer. Such a cocrystal or coformer may be prepared using water-soluble molecules such as saccharin, caffeine, nicotinamide or carboxylic acids. Coformers may be prepared as described in Emami S et al (2018) Biolmpacts 8(4), 305-320.

### N-Oxides

Compounds of the formula (I) containing a nitrogen containing function/moiety may also form N-oxides. A reference herein to a compound of the formula (I) that contains a nitrogen containing function/moiety also includes the N-oxide.

Where a compound contains several nitrogen containing functions/moieties, one or more than one nitrogen atom may be oxidised to form an N-oxide. Particular examples of N-oxides are the N-oxides of a tertiary amine or a nitrogen atom of a nitrogen-containing heterocycle.

N-Oxides can be formed by treatment of the corresponding nitrogen containing function/moiety with an oxidizing agent such as hydrogen peroxide or a per-acid (*e.g.* a peroxycarboxylic acid), see for example Advanced Organic Chemistry, by Jerry March, 4th Edition, Wiley Interscience. More particularly, N-oxides can be made by the procedure of L. W. Deady (Syn. Commun. 1977, 7, 509-514) in which the nitrogen containing function/moiety containingcompound is reacted with *m*-chloroperoxybenzoic acid (mCPBA), for example, in an inert solvent such as dichloromethane.

### Prodrugs

It will be appreciated by those skilled in the art that certain protected derivatives of compounds of formula (I), which may be made prior to a final deprotection stage, may not possess pharmacological activity as such, but may, in certain instances, be administered orally or parenterally and thereafter metabolised in the body to form compounds of the invention which are pharmacologically active. Such derivatives may therefore be described as "prodrugs".

Examples of pro-drug functionality suitable for the compounds of the present invention are described in Drugs of Today, 19, 9, 1983, 499-538 and in Topics in Chemistry, Chapter 31, pp. 306-316 and in *"*Design of Prodrugs" by H. Bundgaard, Elsevier, 1985, Chapter 1.

It will further be appreciated by those skilled in the art, that certain moieties, known to those skilled in the art as "pro-moieties", for example as described by H. Bundgaard in *"Design of Prodrugs"* may be placed on appropriate functionalities when such functionalities are present within compounds of the invention.

Also included within the scope of the compounds of the invention are polymorphs thereof.

### Enantiomers

Where chiral centres are present in compounds of formula (I), the present invention includes within its scope all possible enantiomers and diastereoisomers, including mixtures thereof. The different isomeric forms may be separated or resolved one from the other by conventional methods, or any given isomer may be obtained by conventional synthetic methods or by stereospecific or asymmetric syntheses. The invention also extends to any tautomeric forms or mixtures thereof.

### Isotopes

The subject invention also includes all pharmaceutically acceptable isotopically-labelled compounds which are identical to those recited in formula (I) but for the fact that one or more atoms are replaced by an atom having an atomic mass or mass number different from the atomic mass or mass number most commonly found in nature.

Examples of isotopes suitable for inclusion in the compounds of the invention comprise isotopes of hydrogen, such as ²H (D) and ³H (T), carbon, such as ¹¹C, ¹³C and ¹⁴C, chlorine, such as ³⁶Cl, fluorine, such as ¹⁸F, iodine, such as ¹²³I, ¹²⁵I and ¹³¹I, nitrogen, such as ¹³N and ¹⁵N, oxygen, such as ¹⁵O, ¹⁷O and ¹⁸O, phosphorus, such as ³²P, and sulfur, such as ³⁵S. Certain isotopically-labelled compounds of formula (I), for example, those incorporating a radioactive isotope, are useful in drug and/or substrate tissue distribution studies. The compounds of formula (I) can also have valuable diagnostic properties in that they can be used for detecting or identifying the formation of a complex between a labelled compound and other molecules, peptides, proteins, enzymes or receptors. The detecting or identifying methods can use compounds that are labelled with labelling agents such as radioisotopes, enzymes, fluorescent substances, luminous substances (for example, luminol, luminol derivatives, luciferin, aequorin and luciferase) *etc.* The radioactive isotopes tritium, *i.e.* ³H (T), and carbon-14, *i.e.* ¹⁴C, are particularly useful for this purpose in view of their ease of incorporation and ready means of detection.

Substitution with heavier isotopes such as deuterium, *i.e.* ²H (D), may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining target occupancy.

Isotopically-labelled compounds of formula (I) can generally be prepared by conventional techniques known to those skilled in the art or by processes analogous to those described in the accompanying Examples and Preparations using appropriate isotopically-labelled reagents in place of the non-labelled reagent previously employed.

### Purity

Since the compounds of formula (I) are intended for use in pharmaceutical compositions it will readily be understood that they are each preferably provided in substantially pure form, for example at least 60% pure, more suitably at least 75% pure and preferably at least 85%, especially at least 98% pure (% are given on a weight for weight basis). Impure preparations of the compounds may be used for preparing the more pure forms used in the pharmaceutical compositions.

### Processes

According to a further aspect of the present invention there is provided a process for the preparation of compounds of formula (I) and derivatives thereof. The following schemes are examples of synthetic schemes that may be used to synthesise the compounds of the invention. In the following schemes reactive groups can be protected with protecting groups and de-protected according to well established techniques.

According to a further aspect of the invention there is provided a process for preparing the deuterated derivative of a compound of formula (I) as herein defined which comprises:
(a) reacting a deuterated derivative of a compound of formula (III): 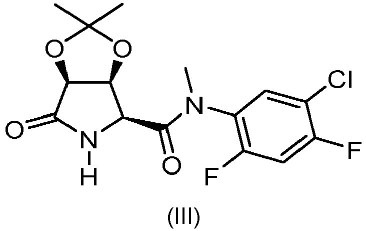 with a compound of formula (IV): 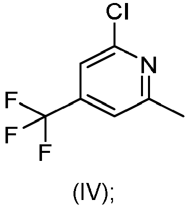
(b) deprotection of a protected derivative of a compound of formula (I); and
(c) interconversion of a compound of formula (I) or protected derivative thereof to a further compound of formula (I) or protected derivative thereof.

Process (a) typically comprises reacting a compound of formula (III) with a compound of formula (IV) in the presence of suitable catalysts and ligands, such as Pd₂(dba)₃ and XantPhos, and in the presence of suitable base, such as K₂CO₃, under suitable conditions, such as heating in a suitable solvent, such as 1,4-dioxane, to a suitable temperature (such as 90-95 °C).

Compounds of formula (III) may be prepared in accordance with the procedures described herein. For example, compounds of formula (III) may be prepared in accordance with the experimental procedure described in Example 1.

In one embodiment, the compound of formula (III) is a compound of formula (III)^{a}:

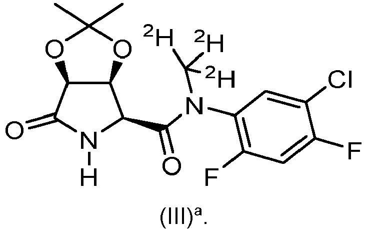

Compounds of formula (IV) are either known or may be prepared in accordance with known procedures.

A wide range of well-known functional group interconversions for process (c) are known by a person skilled in the art for converting a precursor compound to a compound of formula (I) and are described in Advanced Organic Chemistry by Jerry March, 4th Edition, John Wiley & Sons, 1992. For example possible metal catalysed functionalisations such as using organo-tin reagents (the Stille reaction), Grignard reagents and reactions with nitrogen nucleophiles are described in 'Palladium Reagents and Catalysts' [Jiro Tsuji, Wiley, ISBN 0-470-85032-9] and Handbook of OrganoPalladium Chemistry for Organic Synthesis [Volume 1, Edited by Ei-ichi Negishi, Wiley, ISBN 0-471-31506-0].

If appropriate, the reactions described herein are followed or preceded by one or more reactions known to the skilled of the art and are performed in an appropriate order to achieve the requisite substitutions on compounds of formula (I) to afford other compounds of formula (I). Non-limiting examples of such reactions whose conditions can be found in the literature include:
protection of reactive functions,
deprotection of reactive functions,
halogenation,
dehalogenation,
dealkylation,
alkylation of amine, aniline, alcohol and phenol,
Mitsunobu reaction on hydroxyl groups,
cycloaddition reactions on appropriate groups,
reduction of nitro, esters, cyano, aldehydes,
transition metal-catalyzed coupling reactions,
acylation,
sulfonylation/introduction of sulfonyl groups,
saponification/hydrolysis of esters groups,
amidification or transesterification of ester groups,
esterification or amidification of carboxylic groups,
halogen exchange,
nucleophilic substitution with amine, thiol or alcohol,
reductive amination,
oxime formation on carbonyl and hydroxylamine groups,
S-oxidation,
N-oxidation,
salification.

It is recognised that the sequence of reactions involving aryl coupling and reduction may be varied. It is also recognised that a wide range of palladium based catalysts are suitable for conducting aryl coupling reactions.

It may also be recognised that isomer separation may occur at any suitable stage in the synthetic sequence. It should be stressed that such chiral separation forms a key aspect of the invention and that such separation may be conducted in accordance with the methodology described herein or may be conducted in accordance with known methodology. It is also recognised that it may be beneficial to temporarily form a protected derivative of an intermediate in the synthesis, for example, a Boc-protected amine, or SEM-protected amide, in order to facilitate chromatographic separation, chiral resolution or to give improved solubility or yields in particular steps.

In many of the reactions described above, it may be necessary to protect one or more groups to prevent reaction from taking place at an undesirable location on the molecule. Examples of protecting groups, and methods of protecting and de-protecting functional groups, can be found in Protective Groups in Organic Synthesis (T. Green and P. Wuts; 4th Edition; John Wiley and Sons, 2007).

A hydroxy group may be protected, for example, as an ether (-OR) or an ester (-OC(=O)R), for example, as: a *tert*-butyl ether; a tetrahydropyranyl (THP) ether; a benzyl, benzhydryl (diphenylmethyl), or trityl (triphenylmethyl) ether; a trimethylsilyl or *tert*-butyldimethylsilyl ether; or an acetyl ester (-OC(=O)CH₃).

An amine group may be protected, for example, as an amide (-NRCO-R) or a carbamate (-NRCO-OR), for example, as: a methyl amide (-NHCO-CH₃); a benzyl carbamate (-NHCO-OCH₂C₆H₅, -NH-Cbz or NH-Z); as a *tert*-butyl carbamate (-NHCOOC(CH₃)₃, NH-Boc); a 2-biphenyl-2-propyl carbamate (-NHCO-OC(CH₃)₂C₆H₄C₆H₅, NH-Boc), as a 9-fluorenylmethyl carbamate (-NH-Fmoc), as a 6-nitroveratryl carbamate (-NH-Nvoc), as a 2-trimethylsilylethyl carbamate (-NH-Teoc), as a 2,2,2-trichloroethyl carbamate (-NH-Troc), as an allyl carbamate (-NH-Alloc), or as a 2(-phenylsulfonyl)ethyl carbamate (-NH-Psec).

Other protecting groups for amines, such as cyclic amines and heterocyclic N-H groups, include toluenesulfonyl (tosyl) and methanesulfonyl (mesyl) groups, benzyl groups such as a para-methoxybenzyl (PMB) group and tetrahydropyranyl (THP) groups.

A carboxylic acid group may be protected as an ester for example, as: an C₁₋₇ alkyl ester (*e.g.* a methyl ester; a *tert*-butyl ester); a C₁₋₇ haloalkyl ester (*e.g.* a C₁₋₇ trihaloalkyl ester); a triC₁₋₇ alkylsilyl-C₁₋₇ alkyl ester; or a C₅₋₂₀ aryl-C₁₋₇ alkyl ester (*e.g.* a benzyl ester; a nitrobenzyl ester; para-methoxybenzyl ester.

It will be understood by those skilled in the art that certain compounds of the invention can be converted into other compounds of the invention according to standard chemical methods.

### Therapeutic Utility

The compounds of the invention, and examples thereof, are inhibitors of Polθ polymerase activity, and which may be useful in preventing or treating disease states or conditions described herein. In addition, the compounds of the invention will be useful in preventing or treating diseases or condition mediated by Polθ. References to the preventing or prophylaxis or treatment of a disease state or condition such as cancer include within their scope alleviating or reducing the incidence of cancer.

Thus, for example, it is envisaged that the compounds of the invention will be useful in alleviating or reducing the incidence of cancer.

The compounds of the present invention may be useful for the treatment of the adult population. The compounds of the present invention may be useful for the treatment of the pediatric population.

As a consequence of their inhibition of Polθ, the compounds will be useful in providing a means of disabling the ability of cells to perform MMEJ. It is therefore anticipated that the compounds may prove useful in treating or preventing proliferative disorders such as cancers. In addition, the compounds of the invention may be useful in the treatment of diseases in which there is a disorder associated with cell accumulation.

Without being bound by theory it is expected that the Polθ inhibitors of the present invention will demonstrate certain properties for them to be of particular utility in the therapeutic treatment of certain cancers. For example, in one embodiment, the Polθ inhibitors of the present invention are suitably lethal in BRCA1 and BRCA2 deficient primary and secondary solid tumours, including breast, ovarian, prostate and pancreas.

In a further embodiment, the Polθ inhibitors of the present invention are suitably lethal in a variety of primary and secondary solid tumours which are HRD by mechanisms other than BRCA deficiency, such as those with promoter hypermethylation. In these tumours where no DSB repair pathway may be fully down regulated the Polθi may be given along with another DDR modulator such as a PARP inhibitor, a DNA-PK inhibitor, an ATR inhibitor, an ATM inhibitor, a wee1 inhibitor or a CHK1 inhibitor.

In a further embodiment, the Polθ inhibitors of the present invention are suitably lethal in primary and secondary breast, ovarian, prostate and pancreatic tumours retaining BRCA1 deficiency but which, following or not following exposure to PARPi medication, are resistant to PARPi treatment.

In a further embodiment, the Pοlθ inhibitors of the present invention suitably increase the ORR including CRR, will delay the onset of PARPi resistance, will increase the time to relapse and DFS, and will increase the OS of HRD (BRCA1/2 deficient and other HRD mechanisms) primary and secondary tumours (breast, ovarian, prostate and pancreas) when given with PARPi treatment programmes.

In a further embodiment, the Pοlθ inhibitors of the present invention suitably show synthetic sickness and/or synthetic lethality in a variety of tumours with loss of ATM activity (ATM^{-/-}) particularly in the context of WT p53. Tumour types will include around 10% of all solid tumours including gastric, lung, breast, and CRC, along with CLL. Co-medicating with another DDR modifier, such as a DNA-PK inhibitor, PARP inhibitor or ATR inhibitor, may further enhance such activity. Pοlθ inhibitors will resensitise CLL to classical chemotherapy and chemo-immunotherapy where drug resistance has emerged. Thus, according to a further embodiment, the pharmaceutical composition of the present invention additionally comprises a DNA-PK inhibitor, PARP inhibitor or ATR inhibitor.

In a further embodiment, the Pοlθ inhibitors of the present invention suitably show synthetic sickness and/or synthetic lethality in a variety of tumours deficient in the DNA double strand break repair process of non-homologous end-joining (NHEJ-D). Tumour types will include approximately 2-10% of all solid tumours including prostate, pancreatic, cervical, breast, lung, bladder and oesophageal. Co-medicating with another DDR modifier, such as a PARP inhibitor, ATM inhibitor, wee1 inhibitor, CHK inhibitor, or ATR inhibitor, may further enhance such activity. Pοlθ inhibitors will further sensitise NHEJD cancer cells to DNA DSB inducing chemotherapies and to ionising radiation based therapies. Thus, according to a further embodiment, the pharmaceutical composition of the present invention additionally comprises a PARP inhibitor, ATM inhibitor, wee1 inhibitor, CHK inhibitor, or ATR inhibitor.

In a further embodiment, the Pοlθ inhibitors of the present invention suitably reduce the DNA replication stress response during the chemotherapy of HR proficient tumours such as ovarian, NSCL and breast tumours over expressing Polθ. This will increase the ORR to treatment and increase OS. Such effects are particularly likely with cytarabine (Ara-C) and hydroxyurea used in a wide variety of leukemias including CML, and the management of squamous cell carcinomas.

In a further embodiment, the Pοlθ inhibitors of the present invention suitably selectively sensitise solid tumours to radiotherapy, including EBRT and brachytherapy and radioligand based therapies, with little or no sensitisation of normal tissues. In a fractionated curative-intent setting this will increase loco-regional control driving increased survival. This will be particularly evident in the management of NSCLC, SCCH&N, rectal cancer, prostate cancer and pancreatic cancer.

In a further embodiment, the Pοlθ inhibitors of the present invention suitably show synthetic sickness and/or synthetic lethality in PTEN deleted tumours such as CaP, with or without comedication with a PARPi. Furthermore, such tumours will exhibit exquisite sensitivity to radiotherapy both by dint of the PTEN deletion as well as the Polθ inhibitor induced radiosensitivity.

In a further embodiment, the Pοlθ inhibitors of the present invention suitably suppress TLS polymerase activity, sensitising primary and secondary solid tumours (e.g. breast, lung, ovarian, CRC) to drugs (e.g. cisplatin, mitomycin and cyclophosphamide) as well as reducing the acquisition of drug-induced mutations implicated in tumour resistance leading to prolongation of remission and increased TTR.

In a further embodiment, the Pοlθ inhibitors of the present invention suitably resensitise BCR-ABL-positive CML which is has developed imatinib resistance, as well as other solid tumours with elevated ligase IIIα levels, reduced ligase IV levels and increased dependence upon altEJ DSB repair.

In a further embodiment, the Pοlθ inhibitors of the present invention suitably show synthetic sickness and/or synthetic lethality in aromatase inhibitor resistant ER⁻ primary and secondary breast cancers, again showing elevated ligase IIIα levels, reduced ligase IV levels and increased dependence upon altEJ DSB repair.

According to a further aspect of the invention there is a provided a compound of formula (I) as defined herein for use in the treatment of tumours characterised by a deficiency in homologous recombination (HRD).

It will be appreciated that references herein to "deficiency in homologous recombination (HRD)" refer to any genetic variation which results in a deficiency or loss of function of the resultant homologous recombination gene. Examples of said genetic variation include mutations (e.g. point mutations), substitutions, deletions, single nucleotide polymorphisms (SNPs), haplotypes, chromosome abnormalities, Copy Number Variation (CNV), epigenetics, DNA inversions, reduction in expression and mis-localisation.

In one embodiment, said homologous recombination genes are selected from any of: ATM, ATR, BRCA1, BRCA2, BARD1, RAD51C, RAD50, CHEK1, CHEK2, FANCA, FANCB, FANCC, FANCD2, FANCE, FANCF, FANCG, FANCI, FANCL, FANCM, PALB2 (FANCN), FANCP (BTBD12), ERCC4 (FANCQ), PTEN, CDK12, MRE11, NBS1, NBN, CLASPIN, BLM, WRN, SMARCA2, SMARCA4, LIG1, RPA1, RPA2, BRIP1 and PTEN.

It will be appreciated that references herein to "non-homologous end-joining deficiency (NHEJD)" refer to any genetic variation which results in a deficiency or loss of function of the resultant homologous recombination gene. Examples of said genetic variation include mutations (e.g. point mutations), substitutions, deletions, single nucleotide polymorphisms (SNPs), haplotypes, chromosome abnormalities, Copy Number Variation (CNV), epigenetics, DNA inversions, reduction in expression and mis-localisation.

In one embodiment, said non-homologous end-joining genes are selected from any one or more of: LIG4, NHEJ1, POLL, POLM, PRKDC, XRCC4, XRCC5, XRCC6, and DCLRE1C.

According to a further aspect of the invention there is a provided a compound of formula (I) as defined herein for use in the treatment of tumours which overexpress Polθ.

According to a further aspect of the invention there is a provided a compound of formula (I) as defined herein for use in the treatment of tumours which have elevated ligase IIIα levels, reduced ligase IV levels and increased dependence upon altEJ DSB repair.

Examples of cancers (and their benign counterparts) which may be treated (or inhibited) include, but are not limited to tumours of epithelial origin (adenomas and carcinomas of various types including adenocarcinomas, squamous carcinomas, transitional cell carcinomas and other carcinomas) such as carcinomas of the bladder and urinary tract, breast, gastrointestinal tract (including the esophagus, stomach (gastric), small intestine, colon, rectum and anus), liver (hepatocellular carcinoma), gall bladder and biliary system, exocrine pancreas, kidney, lung (for example adenocarcinomas, small cell lung carcinomas, non-small cell lung carcinomas, bronchioalveolar carcinomas and mesotheliomas), head and neck (for example cancers of the tongue, buccal cavity, larynx, pharynx, nasopharynx, tonsil, salivary glands, nasal cavity and paranasal sinuses), ovary, fallopian tubes, peritoneum, vagina, vulva, penis, cervix, myometrium, endometrium, thyroid (for example thyroid follicular carcinoma), adrenal, prostate, skin and adnexae (for example melanoma, basal cell carcinoma, squamous cell carcinoma, keratoacanthoma, dysplastic naevus); haematological malignancies (i.e. leukemias, lymphomas) and premalignant haematological disorders and disorders of borderline malignancy including haematological malignancies and related conditions of lymphoid lineage (for example acute lymphocytic leukemia [ALL], chronic lymphocytic leukemia [CLL], B-cell lymphomas such as diffuse large B-cell lymphoma [DLBCL], follicular lymphoma, Burkitt's lymphoma, mantle cell lymphoma, MALT lymphoma, T-cell lymphomas and leukaemias, natural killer [NK] cell lymphomas, Hodgkin's lymphomas, hairy cell leukaemia, monoclonal gammopathy of uncertain significance, plasmacytoma, multiple myeloma, and post-transplant lymphoproliferative disorders), and haematological malignancies and related conditions of myeloid lineage (for example acute myelogenous leukemia [AML], chronic myelogenous leukemia [CML], chronic myelomonocytic leukemia [CMML], hypereosinophilic syndrome, myeloproliferative disorders such as polycythaemia vera, essential thrombocythaemia and primary myelofibrosis, myeloproliferative syndrome, myelodysplastic syndrome, and promyelocytic leukemia); tumours of mesenchymal origin, for example sarcomas of soft tissue, bone or cartilage such as osteosarcomas, fibrosarcomas, chondrosarcomas, rhabdomyosarcomas, leiomyosarcomas, liposarcomas, angiosarcomas, Kaposi's sarcoma, Ewing's sarcoma, synovial sarcomas, epithelioid sarcomas, gastrointestinal stromal tumours, benign and malignant histiocytomas, and dermatofibrosarcoma protuberans; tumours of the central or peripheral nervous system (for example astrocytomas, gliomas and glioblastomas, meningiomas, ependymomas, pineal tumours and schwannomas); endocrine tumours (for example pituitary tumours, adrenal tumours, islet cell tumours, parathyroid tumours, carcinoid tumours and medullary carcinoma of the thyroid); ocular and adnexal tumours (for example retinoblastoma); germ cell and trophoblastic tumours (for example teratomas, seminomas, dysgerminomas, hydatidiform moles and choriocarcinomas); and paediatric and embryonal tumours (for example medulloblastoma, neuroblastoma, Wilms tumour, and primitive neuroectodermal tumours); or syndromes, congenital or otherwise, which leave the patient susceptible to malignancy (for example Xeroderma Pigmentosum).

Many diseases are characterized by persistent and unregulated angiogenesis. Chronic proliferative diseases are often accompanied by profound angiogenesis, which can contribute to or maintain an inflammatory and/or proliferative state, or which leads to tissue destruction through the invasive proliferation of blood vessels. Tumour growth and metastasis have been found to be angiogenesis-dependent. Compounds of the invention may therefore be useful in preventing and disrupting initiation of tumour angiogenesis. In particular, the compounds of the invention may be useful in the treatment of metastasis and metastatic cancers.

Metastasis or metastatic disease is the spread of a disease from one organ or part to another non-adjacent organ or part. The cancers which can be treated by the compounds of the invention include primary tumours (i.e. cancer cells at the originating site), local invasion (cancer cells which penetrate and infiltrate surrounding normal tissues in the local area), and metastatic (or secondary) tumours ie. tumours that have formed from malignant cells which have circulated through the bloodstream (haematogenous spread) or via lymphatics or across body cavities (trans-coelomic) to other sites and tissues in the body.

Particular cancers include hepatocellular carcinoma, melanoma, oesophageal, renal, colon, colorectal, lung e.g. mesothelioma or lung adenocarcinoma, breast, bladder, gastrointestinal, ovarian and prostate cancers.

A further aspect provides the use of a compound for the manufacture of a medicament for the treatment of a disease or condition as described herein, in particular cancer.

The compounds may also be useful in the treatment of tumour growth, pathogenesis, resistance to chemo- and radio-therapy by sensitising cells to chemotherapy and as an anti-metastatic agent.

The potency of the compounds of the invention as inhibitors of Polθ can be measured using the biological and biophysical assays set forth in the examples herein and the level of affinity exhibited by a given compound can be defined in terms of the IC₅₀ value. Particular compounds of the present invention are compounds having an IC₅₀ value of less than 1 µM, more particularly less than 0.1 µM.

A role for the loss of Pοlθ enhancing the efficacy of CRISPR mediated gene editing has been described in WO 2017/062754. Thus, Pοlθ inhibitory compounds are likely to be useful in enhancing the efficiency of CRISPR based editing methodologies and/or CRISPR based editing therapeutics. Furthermore, compound mediated Pοlθ inhibition is likely to reduce the frequency of random integration events and thus provide a route to ameliorate any safety concerns of CRISPR mediated technology. Thus, according to a further aspect of the invention, there is provided the use of a compound of formula (I) as defined herein in a CRISPR based editing methodology and/or CRISPR based editing therapeutics, such as the enhancement of efficiency of CRISPR based editing methodology and/or CRISPR based editing therapeutics.

### Pharmaceutical Compositions

While it is possible for the active compound to be administered alone, it is preferable to present it as a pharmaceutical composition (e.g. formulation). In one embodiment this is a sterile pharmaceutical composition.

Thus, the present invention further provides pharmaceutical compositions, as defined above, and methods of making a pharmaceutical composition comprising (e.g admixing) at least one compound of formula (I) together with one or more pharmaceutically acceptable excipients and optionally other therapeutic or prophylactic agents, as described herein.

The pharmaceutically acceptable excipient(s) can be selected from, for example, carriers (e.g. a solid, liquid or semi-solid carrier), adjuvants, diluents, fillers or bulking agents, granulating agents, coating agents, release-controlling agents, binding agents, disintegrants, lubricating agents, preservatives, antioxidants, buffering agents, suspending agents, thickening agents, flavouring agents, sweeteners, taste masking agents, stabilisers or any other excipients conventionally used in pharmaceutical compositions. Examples of excipients for various types of pharmaceutical compositions are set out in more detail below.

The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

Pharmaceutical compositions containing compounds of the formula (I) can be formulated in accordance with known techniques, see for example, Remington's Pharmaceutical Sciences, Mack Publishing Company, Easton, PA, USA.

The pharmaceutical compositions can be in any form suitable for oral, parenteral, topical, intranasal, intrabronchial, sublingual, ophthalmic, otic, rectal, intra-vaginal, or transdermal administration. Where the compositions are intended for parenteral administration, they can be formulated for intravenous, intramuscular, intraperitoneal, subcutaneous administration or for direct delivery into a target organ or tissue by injection, infusion or other means of delivery. The delivery can be by bolus injection, short term infusion or longer term infusion and can be via passive delivery or through the utilisation of a suitable infusion pump or syringe driver.

Pharmaceutical formulations adapted for parenteral administration include aqueous and non-aqueous sterile injection solutions which may contain anti-oxidants, buffers, bacteriostats, co-solvents, surface active agents, organic solvent mixtures, cyclodextrin complexation agents, emulsifying agents (for forming and stabilizing emulsion formulations), liposome components for forming liposomes, gellable polymers for forming polymeric gels, lyophilisation protectants and combinations of agents for, *inter alia,* stabilising the active ingredient in a soluble form and rendering the formulation isotonic with the blood of the intended recipient. Pharmaceutical formulations for parenteral administration may also take the form of aqueous and non-aqueous sterile suspensions which may include suspending agents and thickening agents (R. G. Strickly, Solubilizing Excipients in oral and injectable formulations, Pharmaceutical Research, Vol 21(2) 2004, p 201-230).

The formulations may be presented in unit-dose or multi-dose containers, for example sealed ampoules, vials and prefilled syringes, and may be stored in a freeze-dried (lyophilised) condition requiring only the addition of the sterile liquid carrier, for example water for injections, immediately prior to use. In one embodiment, the formulation is provided as an active pharmaceutical ingredient in a bottle for subsequent reconstitution using an appropriate diluent.

The pharmaceutical formulation can be prepared by lyophilising a compound of formula (I), or sub-groups thereof. Lyophilisation refers to the procedure of freeze-drying a composition. Freeze-drying and lyophilisation are therefore used herein as synonyms.

Extemporaneous injection solutions and suspensions may be prepared from sterile powders, granules and tablets.

Pharmaceutical compositions of the present invention for parenteral injection can also comprise pharmaceutically acceptable sterile aqueous or non-aqueous solutions, dispersions, suspensions or emulsions as well as sterile powders for reconstitution into sterile injectable solutions or dispersions just prior to use.

Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, vegetable oils (such as sunflower oil, safflower oil, corn oil or olive oil), and injectable organic esters such as ethyl oleate. Proper fluidity can be maintained, for example, by the use of thickening or coating materials such as lecithin, by the maintenance of the required particle size in the case of dispersions, and by the use of surfactants.

The compositions of the present invention may also contain adjuvants such as preservatives, wetting agents, emulsifying agents, and dispersing agents. Prevention of the action of microorganisms may be ensured by the inclusion of various antibacterial and antifungal agents, for example, paraben, chlorobutanol, phenol, sorbic acid, and the like. It may also be desirable to include agents to adjust tonicity such as sugars, sodium chloride, and the like. Prolonged absorption of the injectable pharmaceutical form may be brought about by the inclusion of agents which delay absorption such as aluminum monostearate and gelatin.

In one particular embodiment of the invention, the pharmaceutical composition is in a form suitable for i.v. administration, for example by injection or infusion. For intravenous administration, the solution can be dosed as is, or can be injected into an infusion bag (containing a pharmaceutically acceptable excipient, such as 0.9% saline or 5% dextrose), before administration.

In another particular embodiment, the pharmaceutical composition is in a form suitable for sub-cutaneous (s.c.) administration.

Pharmaceutical dosage forms suitable for oral administration include tablets (coated or uncoated), capsules (hard or soft shell), caplets, pills, lozenges, syrups, solutions, powders, granules, elixirs and suspensions, sublingual tablets, wafers or patches such as buccal patches.

Thus, tablet compositions can contain a unit dosage of active compound together with an inert diluent or carrier such as a sugar or sugar alcohol, eg; lactose, sucrose, sorbitol or mannitol; and/or a non-sugar derived diluent such as sodium carbonate, calcium phosphate, calcium carbonate, or a cellulose or derivative thereof such as microcrystalline cellulose (MCC), methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Tablets may be designed to release the drug either upon contact with stomach fluids (immediate release tablets) or to release in a controlled manner (controlled release tablets) over a prolonged period of time or with a specific region of the GI tract.

Capsule formulations may be of the hard gelatin or soft gelatin variety and can contain the active component in solid, semi-solid, or liquid form. Gelatin capsules can be formed from animal gelatin or synthetic or plant derived equivalents thereof.

The solid dosage forms (eg; tablets, capsules etc.) can be coated or un-coated. Coatings may act either as a protective film (e.g. a polymer, wax or varnish) or as a mechanism for controlling drug release or for aesthetic or identification purposes. The coating (e.g. a Eudragit ^{™} type polymer) can be designed to release the active component at a desired location within the gastro-intestinal tract. Thus, the coating can be selected so as to degrade under certain pH conditions within the gastrointestinal tract, thereby selectively release the compound in the stomach or in the ileum, duodenum, jejenum or colon.

Instead of, or in addition to, a coating, the drug can be presented in a solid matrix comprising a release controlling agent, for example a release delaying agent which may be adapted to release the compound in a controlled manner in the gastrointestinal tract. Alternatively the drug can be presented in a polymer coating e.g. a polymethacrylate polymer coating, which may be adapted to selectively release the compound under conditions of varying acidity or alkalinity in the gastrointestinal tract. Alternatively, the matrix material or release retarding coating can take the form of an erodible polymer (e.g. a maleic anhydride polymer) which is substantially continuously eroded as the dosage form passes through the gastrointestinal tract. In another alternative, the coating can be designed to disintegrate under microbial action in the gut. As a further alternative, the active compound can be formulated in a delivery system that provides osmotic control of the release of the compound. Osmotic release and other delayed release or sustained release formulations (for example formulations based on ion exchange resins) may be prepared in accordance with methods well known to those skilled in the art.

The compound of formula (I) may be formulated with a carrier and administered in the form of nanoparticles, the increased surface area of the nanoparticles assisting their absorption. In addition, nanoparticles offer the possibility of direct penetration into the cell. Nanoparticle drug delivery systems are described in "Nanoparticle Technology for Drug Delivery", edited by Ram B Gupta and Uday B. Kompella, Informa Healthcare, ISBN 9781574448573, published 13th March 2006. Nanoparticles for drug delivery are also described in J. Control. Release, 2003, 91 (1-2), 167-172, and in Sinha et al., Mol. Cancer Ther. August 1, (2006) 5, 1909.

The pharmaceutical compositions typically comprise from approximately 1% (w/w) to approximately 95% (w/w) active ingredient and from 99% (w/w) to 5% (w/w) of a pharmaceutically acceptable excipient or combination of excipients. Particularly, the compositions comprise from approximately 20% (w/w) to approximately 90% (w/w) active ingredient and from 80% (w/w) to 10% of a pharmaceutically acceptable excipient or combination of excipients. The pharmaceutical compositions comprise from approximately 1% to approximately 95%, particularly from approximately 20% to approximately 90%, active ingredient. Pharmaceutical compositions according to the invention may be, for example, in unit dose form, such as in the form of ampoules, vials, suppositories, pre-filled syringes, dragées, tablets or capsules.

The pharmaceutically acceptable excipient(s) can be selected according to the desired physical form of the formulation and can, for example, be selected from diluents (e.g solid diluents such as fillers or bulking agents; and liquid diluents such as solvents and co-solvents), disintegrants, buffering agents, lubricants, flow aids, release controlling (e.g. release retarding or delaying polymers or waxes) agents, binders, granulating agents, pigments, plasticizers, antioxidants, preservatives, flavouring agents, taste masking agents, tonicity adjusting agents and coating agents.

The skilled person will have the expertise to select the appropriate amounts of ingredients for use in the formulations. For example, tablets and capsules typically contain 0-20% disintegrants, 0-5% lubricants, 0-5% flow aids and/or 0-99% (w/w) fillers/ or bulking agents (depending on drug dose). They may also contain 0-10% (w/w) polymer binders, 0-5% (w/w) antioxidants, 0-5% (w/w) pigments. Slow release tablets would in addition contain 0-99% (w/w) release-controlling (e.g. delaying) polymers (depending on dose). The film coats of the tablet or capsule typically contain 0-10% (w/w) polymers, 0-3% (w/w) pigments, and/or 0-2% (w/w) plasticizers.

Parenteral formulations typically contain 0-20% (w/w) buffers, 0-50% (w/w) cosolvents, and/or 0-99% (w/w) Water for Injection (WFI) (depending on dose and if freeze dried). Formulations for intramuscular depots may also contain 0-99% (w/w) oils.

Pharmaceutical compositions for oral administration can be obtained by combining the active ingredient with solid carriers, if desired granulating a resulting mixture, and processing the mixture, if desired or necessary, after the addition of appropriate excipients, into tablets, dragee cores or capsules. It is also possible for them to be incorporated into a polymer or waxy matrix that allow the active ingredients to diffuse or be released in measured amounts.

The compounds of the invention can also be formulated as solid dispersions. Solid dispersions are homogeneous extremely fine disperse phases of two or more solids. Solid solutions (molecularly disperse systems), one type of solid dispersion, are well known for use in pharmaceutical technology (see (Chiou and Riegelman, J. Pharm. Sci., 60, 1281-1300 (1971)) and are useful in increasing dissolution rates and increasing the bioavailability of poorly water-soluble drugs.

This invention also provides solid dosage forms comprising the solid solution described above. Solid dosage forms include tablets, capsules, chewable tablets and dispersible or effervescent tablets. Known excipients can be blended with the solid solution to provide the desired dosage form. For example, a capsule can contain the solid solution blended with (a) a disintegrant and a lubricant, or (b) a disintegrant, a lubricant and a surfactant. In addition, a capsule can contain a bulking agent, such as lactose or microcrystalline cellulose. A tablet can contain the solid solution blended with at least one disintegrant, a lubricant, a surfactant, a bulking agent and a glidant. A chewable tablet can contain the solid solution blended with a bulking agent, a lubricant, and if desired an additional sweetening agent (such as an artificial sweetener), and suitable flavours. Solid solutions may also be formed by spraying solutions of drug and a suitable polymer onto the surface of inert carriers such as sugar beads ('non-pareils'). These beads can subsequently be filled into capsules or compressed into tablets.

The pharmaceutical formulations may be presented to a patient in "patient packs" containing an entire course of treatment in a single package, usually a blister pack. Patient packs have an advantage over traditional prescriptions, where a pharmacist divides a patient's supply of a pharmaceutical from a bulk supply, in that the patient always has access to the package insert contained in the patient pack, normally missing in patient prescriptions. The inclusion of a package insert has been shown to improve patient compliance with the physician's instructions.

Compositions for topical use and nasal delivery include ointments, creams, sprays, patches, gels, liquid drops and inserts (for example intraocular inserts). Such compositions can be formulated in accordance with known methods.

Examples of formulations for rectal or intra-vaginal administration include pessaries and suppositories which may be, for example, formed from a shaped moldable or waxy material containing the active compound. Solutions of the active compound may also be used for rectal administration.

Compositions for administration by inhalation may take the form of inhalable powder compositions or liquid or powder sprays and can be administrated in standard form using powder inhaler devices or aerosol dispensing devices. Such devices are well known. For administration by inhalation, the powdered formulations typically comprise the active compound together with an inert solid powdered diluent such as lactose.

The compounds of the formula (I) will generally be presented in unit dosage form and, as such, will typically contain sufficient compound to provide a desired level of biological activity. For example, a formulation may contain from 1 nanogram to 2 grams of active ingredient, e.g. from 1 nanogram to 2 milligrams of active ingredient. Within these ranges, particular sub-ranges of compound are 0.1 milligrams to 2 grams of active ingredient (more usually from 10 milligrams to 1 gram, e.g. 50 milligrams to 500 milligrams), or 1 microgram to 20 milligrams (for example 1 microgram to 10 milligrams, e.g. 0.1 milligrams to 2 milligrams of active ingredient).

For oral compositions, a unit dosage form may contain from 1 milligram to 2 grams, more typically 10 milligrams to 1 gram, for example 50 milligrams to 1 gram, e.g. 100 miligrams to 1 gram, of active compound.

The active compound will be administered to a patient in need thereof (for example a human or animal patient) in an amount sufficient to achieve the desired therapeutic effect.

### Methods of Treatment

The compounds of the formula (I) may be useful in the prophylaxis or treatment of a range of disease states or conditions mediated by Polθ. Thus, according to a further aspect of the disclosure there is provided a method of treating a disease state or condition mediated by Pοlθ (e.g. cancer) which comprises administering to a subject in need thereof a compound of formula (I) as described herein. Examples of such disease states and conditions are set out above, and in particular include cancer.

The compounds are generally administered to a subject in need of such administration, for example a human or animal patient, particularly a human.

The compounds will typically be administered in amounts that are therapeutically or prophylactically useful and which generally are non-toxic. However, in certain situations (for example in the case of life threatening diseases), the benefits of administering a compound of the formula (I) may outweigh the disadvantages of any toxic effects or side effects, in which case it may be considered desirable to administer compounds in amounts that are associated with a degree of toxicity.

The compounds may be administered over a prolonged term to maintain beneficial therapeutic effects or may be administered for a short period only. Alternatively they may be administered in a continuous manner or in a manner that provides intermittent dosing (e.g. a pulsatile manner).

A typical daily dose of the compound of formula (I) can be in the range from 100 picograms to 100 milligrams per kilogram of body weight, more typically 5 nanograms to 25 milligrams per kilogram of bodyweight, and more usually 10 nanograms to 15 milligrams per kilogram (e.g. 10 nanograms to 10 milligrams, and more typically 1 microgram per kilogram to 20 milligrams per kilogram, for example 1 microgram to 10 milligrams per kilogram) per kilogram of bodyweight although higher or lower doses may be administered where required. The compound of the formula (I) can be administered on a daily basis or on a repeat basis every 2, or 3, or 4, or 5, or 6, or 7, or 10 or 14, or 21, or 28 days for example.

The compounds of the invention may be administered orally in a range of doses, for example 1 to 1500 mg, 2 to 800 mg, or 5 to 500 mg, e.g. 2 to 200 mg or 10 to 1000 mg, particular examples of doses including 10, 20, 50 and 80 mg. The compound may be administered once or more than once each day, for example one suitable dosage regime may require 1000 mg to 1500 mg two or three times per day. The compound can be administered continuously (i.e. taken every day without a break for the duration of the treatment regimen). Alternatively, the compound can be administered intermittently (i.e. taken continuously for a given period such as a week, then discontinued for a period such as a week and then taken continuously for another period such as a week and so on throughout the duration of the treatment regimen). Examples of treatment regimens involving intermittent administration include regimens wherein administration is in cycles of one week on, one week off; or two weeks on, one week off; or three weeks on, one week off; or two weeks on, two weeks off; or four weeks on two weeks off; or one week on three weeks off - for one or more cycles, e.g. 2, 3, 4, 5, 6, 7, 8, 9 or 10 or more cycles.

In one particular dosing schedule, a patient will be given an infusion of a compound of the formula (I) for periods of one hour daily for up to ten days in particular up to five days for one week, and the treatment repeated at a desired interval such as two to four weeks, in particular every three weeks.

More particularly, a patient may be given an infusion of a compound of the formula (I) for periods of one hour daily for 5 days and the treatment repeated every three weeks.

In another particular dosing schedule, a patient is given an infusion over 30 minutes to 1 hour followed by maintenance infusions of variable duration, for example 1 to 5 hours, e.g. 3 hours.

In a further particular dosing schedule, a patient is given a continuous infusion for a period of 12 hours to 5 days, an in particular a continuous infusion of 24 hours to 72 hours.

In another particular dosing schedule, a patient is given the compound orally once a week.

In another particular dosing schedule, a patient is given the compound orally once-daily for between 7 and 28 days such as 7, 14 or 28 days.

In another particular dosing schedule, a patient is given the compound orally once-daily for 1 day, 2 days, 3 days, 5 days or 1 week followed by the required amount of days off to complete a one or two week cycle.

In another particular dosing schedule, a patient is given the compound orally once-daily for 2 weeks followed by 2 weeks off.

In another particular dosing schedule, a patient is given the compound orally once-daily for 2 weeks followed by 1 week off.

In another particular dosing schedule, a patient is given the compound orally once-daily for 1 week followed by 1 week off.

Ultimately, however, the quantity of compound administered and the type of composition used will be commensurate with the nature of the disease or physiological condition being treated and will be at the discretion of the physician.

It will be appreciated that Pοlθ inhibitors can be used as a single agent or in combination with other anticancer agents. Combination experiments can be performed, for example, as described in Chou TC, Talalay P. Quantitative analysis of dose-effect relationships: the combined effects of multiple drugs or enzyme inhibitors. Adv Enzyme Regulat 1984;22: 27-55.

The compounds as defined herein can be administered as the sole therapeutic agent or they can be administered in combination therapy with one of more other compounds (or therapies) for treatment of a particular disease state, for example a neoplastic disease such as a cancer as hereinbefore defined. For the treatment of the above conditions, the compounds of the invention may be advantageously employed in combination with one or more other medicinal agents, more particularly, with other anti-cancer agents or adjuvants (supporting agents in the therapy) in cancer therapy. Examples of other therapeutic agents or treatments that may be administered together (whether concurrently or at different time intervals) with the compounds of the formula (I) include but are not limited to:
- Topoisomerase I inhibitors;
- Antimetabolites;
- Tubulin targeting agents;
- DNA binder and topoisomerase II inhibitors;
- Alkylating Agents;
- Monoclonal Antibodies;
- Anti-Hormones;
- Signal Transduction Inhibitors;
- Proteasome Inhibitors;
- DNA methyl transferase inhibitors;
- Cytokines and retinoids;
- Chromatin targeted therapies;
- Radiotherapy; and
- Other therapeutic or prophylactic agents.

Particular examples of anti-cancer agents or adjuvants (or salts thereof), include but are not limited to any of the agents selected from groups (i)-(xlvi), and optionally group (xlvii), below:
(i) Platinum compounds, for example cisplatin (optionally combined with amifostine), carboplatin or oxaliplatin;
(ii) Taxane compounds, for example paclitaxel, paclitaxel protein bound particles (Abraxane^{™}), docetaxel, cabazitaxel or larotaxel;
(iii) Topoisomerase I inhibitors, for example camptothecin compounds, for example camptothecin, irinotecan(CPT11), SN-38, or topotecan;
(iv) Topoisomerase II inhibitors, for example anti-tumour epipodophyllotoxins or podophyllotoxin derivatives for example etoposide, or teniposide;
(v) Vinca alkaloids, for example vinblastine, vincristine, liposomal vincristine (Onco-TCS), vinorelbine, vindesine, vinflunine or vinvesir;
(vi) Nucleoside derivatives, for example 5-fluorouracil (5-FU, optionally in combination with leucovorin), gemcitabine, capecitabine, tegafur, UFT, S1, cladribine, cytarabine (Ara-C, cytosine arabinoside), fludarabine, clofarabine, or nelarabine;
(vii) Antimetabolites, for example clofarabine, aminopterin, or methotrexate, azacitidine, cytarabine, floxuridine, pentostatin, thioguanine, thiopurine, 6-mercaptopurine, or hydroxyurea (hydroxycarbamide);
(viii) Alkylating agents, such as nitrogen mustards or nitrosourea, for example cyclophosphamide, chlorambucil, carmustine (BCNU), bendamustine, thiotepa, melphalan, treosulfan, lomustine (CCNU), altretamine, busulfan, dacarbazine, estramustine, fotemustine, ifosfamide (optionally in combination with mesna), pipobroman, procarbazine, streptozocin, temozolomide, uracil, mechlorethamine, methylcyclohexylchloroethylnitrosurea, or nimustine (ACNU);
(ix) Anthracyclines, anthracenediones and related drugs, for example daunorubicin, doxorubicin (optionally in combination with dexrazoxane), liposomal formulations of doxorubicin (eg. Caelyx^{™}, Myocet^{™}, Doxil^{™}), idarubicin, mitoxantrone, epirubicin, amsacrine, or valrubicin;
(x) Epothilones, for example ixabepilone, patupilone, BMS-310705, KOS-862 and ZK-EPO, epothilone A, epothilone B, desoxyepothilone B (also known as epothilone D or KOS-862), aza-epothilone B (also known as BMS-247550), aulimalide, isolaulimalide, or luetherobin;
(xi) DNA methyl transferase inhibitors, for example temozolomide, azacytidine or decitabine, or SGI-110;
(xii) Antifolates, for example methotrexate, pemetrexed disodium, or raltitrexed;
(xiii) Cytotoxic antibiotics, for example antinomycin D, bleomycin, mitomycin C, dactinomycin, carminomycin, daunomycin, levamisole, plicamycin, or mithramycin;
(xiv) Tubulin-binding agents, for example combrestatin, colchicines or nocodazole;
(xv) Signal Transduction inhibitors such as Kinase inhibitors (e.g. EGFR (epithelial growth factor receptor) inhibitors, VEGFR (vascular endothelial growth factor receptor) inhibitors, PDGFR (platelet-derived growth factor receptor) inhibitors, MTKI (multi target kinase inhibitors), Raf inhibitors, mTOR inhibitors for example imatinib mesylate, erlotinib, gefitinib, dasatinib, lapatinib, dovotinib, axitinib, nilotinib, vandetanib, vatalinib, pazopanib, sorafenib, sunitinib, temsirolimus, everolimus (RAD 001), vemurafenib (PLX4032/RG7204), dabrafenib, encorafenib or an IκB kinase inhibitor such as SAR-113945, bardoxolone, BMS-066, BMS-345541, IMD-0354, IMD-2560, or IMD-1041, or MEK inhibitors such as Selumetinib (AZD6244) and Trametinib (GSK121120212);
(xvi) Aurora kinase inhibitors for example AT9283, barasertib (AZD1152), TAK-901, MK0457 (VX680), cenisertib (R-763), danusertib (PHA-739358), alisertib (MLN-8237), or MP-470;
(xvii) CDK inhibitors for example AT7519, roscovitine, seliciclib, alvocidib (flavopiridol), dinaciclib (SCH-727965), 7-hydroxy-staurosporine (UCN-01), JNJ-7706621, BMS-387032 (a.k.a. SNS-032), PHA533533, PD332991, ZK-304709, or AZD-5438;
(xviii) PKA/B inhibitors and PKB (akt) pathway inhibitors for example AKT inhibitors such as KRX-0401 (perifosine/ NSC 639966), ipatasertib (GDC-0068; RG-7440), afuresertib (GSK-2110183; 2110183), MK-2206, MK-8156, AT13148, AZD-5363, triciribine phosphate (VQD-002; triciribine phosphate monohydrate (API-2; TCN-P; TCN-PM; VD-0002), RX-0201, NL-71-101, SR-13668, PX-316, AT13148, AZ-5363, Semaphore, SF1126, or Enzastaurin HCl (LY317615) or MTOR inhibitors such as rapamycin analogues such as RAD 001 (everolimus), CCI 779 (temsirolemus), AP23573 and ridaforolimus, sirolimus (originally known as rapamycin), AP23841 and AP23573, calmodulin inhibitors e.g. CBP-501 (forkhead translocation inhibitors), enzastaurin HCl (LY317615) or PI3K Inhibitors such as dactolisib (BEZ235), buparlisib (BKM-120; NVP-BKM-120), BYL719, copanlisib (BAY-80-6946), ZSTK-474, CUDC-907, apitolisib (GDC-0980; RG-7422), pictilisib (pictrelisib, GDC-0941, RG-7321), GDC-0032, GDC-0068, GSK-2636771, idelalisib (formerly CAL-101, GS 1101, GS-1101), MLN1117 (INK1117), MLN0128 (INK128), IPI-145 (INK1197), LY-3023414, ipatasertib, afuresertib, MK-2206, MK-8156, LY-3023414, LY294002, SF1126 or PI-103, or sonolisib (PX-866);
(xix) Hsp90 inhibitors for example AT13387, herbimycin, geldanamycin (GA), 17-allylamino-17-desmethoxygeldanamycin (17-AAG) e.g. NSC-330507, Kos-953 and CNF-1010, 17-dimethylaminoethylamino-17-demethoxygeldanamycin hydrochloride (17-DMAG) e.g. NSC-707545 and Kos-1022, NVP-AUY922 (VER-52296), NVP-BEP800, CNF-2024 (BIIB-021 an oral purine), ganetespib (STA-9090), SNX-5422 (SC-102112) or IPI-504;
(xx) Monoclonal Antibodies (unconjugated or conjugated to radioisotopes, toxins or other agents), antibody derivatives and related agents, such as anti-CD, anti-VEGFR, anti-HER2, anti-CTLA4, anti-PD-1 or anti-EGFR antibodies, for example rituximab (CD20), ofatumumab (CD20), ibritumomab tiuxetan (CD20), GA101 (CD20), tositumomab (CD20), epratuzumab (CD22), lintuzumab (CD33), gemtuzumab ozogamicin (CD33), alemtuzumab (CD52), galiximab (CD80), trastuzumab (HER2 antibody), pertuzumab (HER2), trastuzumab-DM1 (HER2), ertumaxomab (HER2 and CD3), cetuximab (EGFR), panitumumab (EGFR), necitumumab (EGFR), nimotuzumab (EGFR), bevacizumab (VEGF), catumaxumab (EpCAM and CD3), abagovomab (CA125), farletuzumab (folate receptor), elotuzumab (CS1), denosumab (RANK ligand), figitumumab (IGF1R), CP751,871 (IGF1R), mapatumumab (TRAIL receptor), metMAB (met), mitumomab (GD3 ganglioside), naptumomab estafenatox (5T4), siltuximab (IL6), or immunomodulating agents such as CTLA-4 blocking antibodies and/or antibodies against PD-1 and PD-L1 and/or PD-L2 for example ipilimumab (CTLA4), MK-3475 (pembrolizumab, formerly lambrolizumab, anti-PD-1), nivolumab (anti-PD-1), BMS-936559 (anti- PD-L1), MPDL320A, AMP-514 or MEDI4736 (anti-PD-L1), or tremelimumab (formerly ticilimumab, CP-675,206, anti-CTLA-4);
(xxi) Estrogen receptor antagonists or selective estrogen receptor modulators (SERMs) or inhibitors of estrogen synthesis, for example tamoxifen, fulvestrant, toremifene, droloxifene, faslodex, or raloxifene;
(xxii) Aromatase inhibitors and related drugs, such as exemestane, anastrozole, letrazole, testolactone aminoglutethimide, mitotane or vorozole;
(xxiii) Antiandrogens (i.e. androgen receptor antagonists) and related agents for example bicalutamide, nilutamide, flutamide, cyproterone, or ketoconazole;
(xxiv) Hormones and analogues thereof such as medroxyprogesterone, diethylstilbestrol (a.k.a. diethylstilboestrol) or octreotide;
(xxv) Steroids for example dromostanolone propionate, megestrol acetate, nandrolone (decanoate, phenpropionate), fluoxymestrone or gossypol,
(xxvi) Steroidal cytochrome P450 17alpha-hydroxylase-17,20-lyase inhibitor (CYP17), e.g. abiraterone;
(xxvii) Gonadotropin releasing hormone agonists or antagonists (GnRAs) for example abarelix, goserelin acetate, histrelin acetate, leuprolide acetate, triptorelin, buserelin, or deslorelin;
(xxviii) Glucocorticoids, for example prednisone, prednisolone, dexamethasone;
(xxix) Differentiating agents, such as retinoids, rexinoids, vitamin D or retinoic acid and retinoic acid metabolism blocking agents (RAMBA) for example accutane, alitretinoin, bexarotene, or tretinoin;
(xxx) Farnesyltransferase inhibitors for example tipifarnib;
(xxxi) Chromatin targeted therapies such as histone deacetylase (HDAC) inhibitors for example panobinostat, resminostat, abexinostat, vorinostat, romidepsin, belinostat, entinostat, quisinostat, pracinostat, tefinostat, mocetinostat, givinostat, CUDC-907, CUDC-101, ACY-1215, MGCD-290, EVP-0334, RG-2833, 4SC-202, romidepsin, AR-42 (Ohio State University), CG-200745, valproic acid, CKD-581, sodium butyrate, suberoylanilide hydroxamide acid (SAHA), depsipeptide (FR 901228), dacinostat (NVP-LAQ824), R306465/ JNJ-16241199, JNJ-26481585, trichostatin A, chlamydocin, A-173, JNJ-MGCD-0103, PXD-101, or apicidin;
(xxxii) Proteasome Inhibitors for example bortezomib, carfilzomib, delanzomib (CEP-18770), ixazomib (MLN-9708), oprozomib (ONX-0912) or marizomib;
(xxxiii) Photodynamic drugs for example porfimer sodium or temoporfin;
(xxxiv) Marine organism-derived anticancer agents such as trabectidin;
(xxxv) Radiolabelled drugs for radioimmunotherapy for example with a beta particle-emitting isotope (e.g. Iodine -131, Yittrium -90) or an alpha particle-emitting isotope (e.g., Bismuth-213 or Actinium-225) for example ibritumomab or Iodine tositumomab;
(xxxvi) Telomerase inhibitors for example telomestatin;
(xxxvii) Matrix metalloproteinase inhibitors for example batimastat, marimastat, prinostat or metastat;
(xxxviii) Recombinant interferons (such as interferon-γ and interferon α) and interleukins (e.g. interleukin 2), for example aldesleukin, denileukin diftitox, interferon alfa 2a, interferon alfa 2b, or peginterferon alfa 2b;
(xxxix) Selective immunoresponse modulators for example thalidomide, or lenalidomide; (xl) Therapeutic Vaccines such as sipuleucel-T (Provenge) or OncoVex;
(xli) Cytokine-activating agents include Picibanil, Romurtide, Sizofiran, Virulizin, or Thymosin;
(xlii) Arsenic trioxide;
(xliii) Inhibitors of G-protein coupled receptors (GPCR) for example atrasentan;
(xliv) Enzymes such as L-asparaginase, pegaspargase, rasburicase, or pegademase;
(xlv) DNA repair inhibitors such as PARP inhibitors for example, olaparib, velaparib, iniparib, rucaparib (AG-014699 or PF-01367338), talazoparib or AG-014699;
(xlvi) DNA damage response inhibitors such as ATM inhibitors AZD0156 MS3541, ATR inhibitors AZD6738, M4344, M6620 wee1 inhibitor AZD1775;
(xlvii) Agonists of Death receptor (e.g. TNF-related apoptosis inducing ligand (TRAIL) receptor), such as mapatumumab (formerly HGS-ETR1), conatumumab (formerly AMG 655), PRO95780, lexatumumab, dulanermin, CS-1008, apomab or recombinant TRAIL ligands such as recombinant Human TRAIL/Apo2 Ligand;
(xlviii) Prophylactic agents (adjuncts); i.e. agents that reduce or alleviate some of the side effects associated with chemotherapy agents, for example
   - anti-emetic agents,
   - agents that prevent or decrease the duration of chemotherapy-associated neutropenia and prevent complications that arise from reduced levels of platelets, red blood cells or white blood cells, for example interleukin-11 (e.g. oprelvekin), erythropoietin (EPO) and analogues thereof (e.g. darbepoetin alfa), colony-stimulating factor analogs such as granulocyte macrophage-colony stimulating factor (GM-CSF) (e.g. sargramostim), and granulocyte-colony stimulating factor (G-CSF) and analogues thereof (e.g. filgrastim, pegfilgrastim),
   - agents that inhibit bone resorption such as denosumab or bisphosphonates e.g. zoledronate, zoledronic acid, pamidronate and ibandronate,
   - agents that suppress inflammatory responses such as dexamethasone, prednisone, and prednisolone,
   - agents used to reduce blood levels of growth hormone and IGF-I (and other hormones) in patients with acromegaly or other rare hormone-producing tumours, such as synthetic forms of the hormone somatostatin e.g. octreotide acetate,
   - antidote to drugs that decrease levels of folic acid such as leucovorin, or folinic acid,
   - agents for pain e.g. opiates such as morphine, diamorphine and fentanyl,
   - non-steroidal anti-inflammatory drugs (NSAID) such as COX-2 inhibitors for example celecoxib, etoricoxib and lumiracoxib,
   - agents for mucositis e.g. palifermin,
   - agents for the treatment of side-effects including anorexia, cachexia, oedema or thromoembolic episodes, such as megestrol acetate.

In one embodiment the anticancer is selected from recombinant interferons (such as interferon-γ and interferon α) and interleukins (e.g. interleukin 2), for example aldesleukin, denileukin diftitox, interferon alfa 2a, interferon alfa 2b, or peginterferon alfa 2b; interferon-α2 (500 µ/ml) in particular interferon-β; and signal transduction inhibitors such as kinase inhibitors (e.g. EGFR (epithelial growth factor receptor) inhibitors, VEGFR (vascular endothelial growth factor receptor) inhibitors, PDGFR (platelet-derived growth factor receptor) inhibitors, MTKI (multi target kinase inhibitors), Raf inhibitors, mTOR inhibitors for example imatinib mesylate, erlotinib, gefitinib, dasatinib, lapatinib, dovotinib, axitinib, nilotinib, vandetanib, vatalinib, pazopanib, sorafenib, sunitinib, temsirolimus, everolimus (RAD 001), vemurafenib (PLX4032/RG7204), dabrafenib, encorafenib or an IκB kinase inhibitor such as SAR-113945, bardoxolone, BMS-066, BMS-345541, IMD-0354, IMD-2560, or IMD-1041, or MEK inhibitors such as Selumetinib (AZD6244) and Trametinib (GSK121120212), in particular Raf inhibitors (e.g. vemurafenib) or MEK inhibitors (e.g. trametinib).

Each of the compounds present in the combinations of the invention may be given in individually varying dose schedules and via different routes. As such, the posology of each of the two or more agents may differ: each may be administered at the same time or at different times. A person skilled in the art would know through his or her common general knowledge the dosing regimes and combination therapies to use. For example, the compound of the invention may be using in combination with one or more other agents which are administered according to their existing combination regimen. Examples of standard combination regimens are provided below.

The taxane compound is advantageously administered in a dosage of 50 to 400 mg per square meter (mg/m²) of body surface area, for example 75 to 250 mg/m², particularly for paclitaxel in a dosage of about 175 to 250 mg/m² and for docetaxel in about 75 to 150 mg/m² per course of treatment.

The camptothecin compound is advantageously administered in a dosage of 0.1 to 400 mg per square meter (mg/m²) of body surface area, for example 1 to 300 mg/m², particularly for irinotecan in a dosage of about 100 to 350 mg/m² and for topotecan in about 1 to 2 mg/m² per course of treatment.

The anti-tumour podophyllotoxin derivative is advantageously administered in a dosage of 30 to 300 mg per square meter (mg/m²) of body surface area, for example 50 to 250mg/m², particularly for etoposide in a dosage of about 35 to 100 mg/m² and for teniposide in about 50 to 250 mg/m² per course of treatment.

The anti-tumour vinca alkaloid is advantageously administered in a dosage of 2 to 30 mg per square meter (mg/m²) of body surface area, particularly for vinblastine in a dosage of about 3 to 12 mg/m², for vincristine in a dosage of about 1 to 2 mg/m², and for vinorelbine in dosage of about 10 to 30 mg/m² per course of treatment.

The anti-tumour nucleoside derivative is advantageously administered in a dosage of 200 to 2500 mg per square meter (mg/m²) of body surface area, for example 700 to 1500 mg/m², particularly for 5-FU in a dosage of 200 to 500mg/m², for gemcitabine in a dosage of about 800 to 1200 mg/m² and for capecitabine in about 1000 to 2500 mg/m² per course of treatment.

The alkylating agents such as nitrogen mustard or nitrosourea is advantageously administered in a dosage of 100 to 500 mg per square meter (mg/m²) of body surface area, for example 120 to 200 mg/m², particularly for cyclophosphamide in a dosage of about 100 to 500 mg/m², for chlorambucil in a dosage of about 0.1 to 0.2 mg/kg, for carmustine in a dosage of about 150 to 200 mg/m², and for lomustine in a dosage of about 100 to 150 mg/m² per course of treatment.

The anti-tumour anthracycline derivative is advantageously administered in a dosage of 10 to 75 mg per square meter (mg/m²) of body surface area, for example 15 to 60 mg/m², particularly for doxorubicin in a dosage of about 40 to 75 mg/m², for daunorubicin in a dosage of about 25 to 45mg/m², and for idarubicin in a dosage of about 10 to 15 mg/m² per course of treatment.

The antiestrogen agent is advantageously administered in a dosage of about 1 to 100 mg daily depending on the particular agent and the condition being treated. Tamoxifen is advantageously administered orally in a dosage of 5 to 50 mg, particularly 10 to 20 mg twice a day, continuing the therapy for sufficient time to achieve and maintain a therapeutic effect. Toremifene is advantageously administered orally in a dosage of about 60mg once a day, continuing the therapy for sufficient time to achieve and maintain a therapeutic effect. Anastrozole is advantageously administered orally in a dosage of about 1mg once a day. Droloxifene is advantageously administered orally in a dosage of about 20-100mg once a day. Raloxifene is advantageously administered orally in a dosage of about 60mg once a day. Exemestane is advantageously administered orally in a dosage of about 25mg once a day.

Antibodies are advantageously administered in a dosage of about 1 to 5 mg per square meter (mg/m²) of body surface area, or as known in the art, if different. Trastuzumab is advantageously administered in a dosage of 1 to 5 mg per square meter (mg/m²) of body surface area, particularly 2 to 4mg/m² per course of treatment.

Where the compound of the formula (I) is administered in combination therapy with one, two, three, four or more other therapeutic agents (particularly one or two, more particularly one), the compounds can be administered simultaneously or sequentially. In the latter case, the two or more compounds will be administered within a period and in an amount and manner that is sufficient to ensure that an advantageous or synergistic effect is achieved. When administered sequentially, they can be administered at closely spaced intervals (for example over a period of 5-10 minutes) or at longer intervals (for example 1, 2, 3, 4 or more hours apart, or even longer periods apart where required), the precise dosage regimen being commensurate with the properties of the therapeutic agent(s). These dosages may be administered for example once, twice or more per course of treatment, which may be repeated for example every 7, 14, 21 or 28 days.

In one embodiment is provided a compound of formula (I) for the manufacture of a medicament for use in therapy wherein said compound is used in combination with one, two, three, or four other therapeutic agents. In another embodiment is provided a medicament for treating cancer which comprises a compound of formula (I) wherein said medicament is used in combination with one, two, three, or four other therapeutic agents. The invention further provides use of a compound of formula (I) for the manufacture of a medicament for enhancing or potentiating the response rate in a patient suffering from a cancer where the patient is being treated with one, two, three, or four other therapeutic agents.

It will be appreciated that the particular method and order of administration and the respective dosage amounts and regimes for each component of the combination will depend on the particular other medicinal agent and compound of the present invention being administered, their route of administration, the particular tumour being treated and the particular host being treated. The optimum method and order of administration and the dosage amounts and regime can be readily determined by those skilled in the art using conventional methods and in view of the information set out herein.

The weight ratio of the compound according to the present invention and the one or more other anticancer agent(s) when given as a combination may be determined by the person skilled in the art. Said ratio and the exact dosage and frequency of administration depends on the particular compound according to the invention and the other anticancer agent(s) used, the particular condition being treated, the severity of the condition being treated, the age, weight, gender, diet, time of administration and general physical condition of the particular patient, the mode of administration as well as other medication the individual may be taking, as is well known to those skilled in the art. Furthermore, it is evident that the effective daily amount may be lowered or increased depending on the response of the treated subject and/or depending on the evaluation of the physician prescribing the compounds of the instant invention. A particular weight ratio for the present compound of formula (I) and another anticancer agent may range from 1/10 to 10/1, more in particular from 1/5 to 5/1, even more in particular from 1/3 to 3/1.

The compounds of the invention may also be administered in conjunction with non-chemotherapeutic treatments such as radiotherapy, photodynamic therapy, gene therapy; surgery and controlled diets.

The compounds of the present invention also have therapeutic applications in sensitising tumour cells for radiotherapy and chemotherapy. Hence the compounds of the present invention can be used as "radiosensitizer" and/or "chemosensitizer" or can be given in combination with another "radiosensitizer" and/or "chemosensitizer". In one embodiment the compound of the invention is for use as chemosensitiser.

The term "radiosensitizer" is defined as a molecule administered to patients in therapeutically effective amounts to increase the sensitivity of the cells to ionizing radiation and/or to promote the treatment of diseases which are treatable with ionizing radiation.

The term "chemosensitizer" is defined as a molecule administered to patients in therapeutically effective amounts to increase the sensitivity of cells to chemotherapy and/or promote the treatment of diseases which are treatable with chemotherapeutics.

In one embodiment the compound of the invention is administered with a "radiosensitizer" and/or "chemosensitizer". In one embodiment the compound of the invention is administered with an "immune sensitizer".

The term "immune sensitizer" is defined as a molecule administered to patients in therapeutically effective amounts to increase the sensitivity of cells to a Polθ inhibitor.

Many cancer treatment protocols currently employ radiosensitizers in conjunction with radiation of x-rays. Examples of x-ray activated radiosensitizers include, but are not limited to, the following: metronidazole, misonidazole, desmethylmisonidazole, pimonidazole, etanidazole, nimorazole, mitomycin C, RSU 1069, SR 4233, EO9, RB 6145, nicotinamide, 5-bromodeoxyuridine (BUdR), 5- iododeoxyuridine (IUdR), bromodeoxycytidine, fluorodeoxyuridine (FudR), hydroxyurea, cisplatin, and therapeutically effective analogs and derivatives of the same.

Photodynamic therapy (PDT) of cancers employs visible light as the radiation activator of the sensitizing agent. Examples of photodynamic radiosensitizers include the following, but are not limited to: hematoporphyrin derivatives, Photofrin, benzoporphyrin derivatives, tin etioporphyrin, pheoborbide-a, bacteriochlorophyll-a, naphthalocyanines, phthalocyanines, zinc phthalocyanine, and therapeutically effective analogs and derivatives of the same.

Radiosensitizers may be administered in conjunction with a therapeutically effective amount of one or more other compounds, including but not limited to: compounds of the invention; compounds which promote the incorporation of radiosensitizers to the target cells; compounds which control the flow of therapeutics, nutrients, and/or oxygen to the target cells; chemotherapeutic agents which act on the tumour with or without additional radiation; or other therapeutically effective compounds for treating cancer or other diseases.

Chemosensitizers may be administered in conjunction with a therapeutically effective amount of one or more other compounds, including but not limited to: compounds of the invention; compounds which promote the incorporation of chemosensitizers to the target cells; compounds which control the flow of therapeutics, nutrients, and/or oxygen to the target cells; chemotherapeutic agents which act on the tumour or other therapeutically effective compounds for treating cancer or other disease. Calcium antagonists, for example verapamil, are found useful in combination with antineoplastic agents to establish chemosensitivity in tumor cells resistant to accepted chemotherapeutic agents and to potentiate the efficacy of such compounds in drug-sensitive malignancies.

Examples of immune sensitizers include the following, but are not limited to: immunomodulating agents, for example monoclonal antibodies such as immune checkpoint antibodies [e.g. CTLA-4 blocking antibodies and/or antibodies against PD-1 and PD-L1 and/or PD-L2 for example ipilimumab (CTLA4), MK-3475 (pembrolizumab, formerly lambrolizumab, anti-PD-1), nivolumab (anti-PD-1), BMS-936559 (anti- PD-L1), MPDL320A, AMP-514 or MEDI4736 (anti-PD-L1), or tremelimumab (formerly ticilimumab, CP-675,206, anti-CTLA-4)]; or Signal Transduction inhibitors; or cytokines (such as recombinant interferons); or oncolytic viruses; or immune adjuvants (e.g. BCG).

Immune sensitizers may be administered in conjunction with a therapeutically effective amount of one or more other compounds, including but not limited to: compounds of the invention; compounds which promote the incorporation of immune sensitizers to the target cells; compounds which control the flow of therapeutics, nutrients, and/or oxygen to the target cells; therapeutic agents which act on the tumour or other therapeutically effective compounds for treating cancer or other disease.

For use in combination therapy with another chemotherapeutic agent, the compound of the formula (I) and one, two, three, four or more other therapeutic agents can be, for example, formulated together in a dosage form containing two, three, four or more therapeutic agents i.e. in a unitary pharmaceutical composition containing all agents. In an alternative embodiment, the individual therapeutic agents may be formulated separately and presented together in the form of a kit, optionally with instructions for their use.

In one embodiment is provided a combination of a compound of formula (I) with one or more (e.g. 1 or 2) other therapeutic agents (e.g. anticancer agents as described above). In a further embodiment is provided a combination of a Pοlθ inhibitor as described herein and a PI3K/AKT pathway inhibitor selected from: apitolisib, buparlisib, Copanlisib, pictilisib, ZSTK-474, CUDC-907, GSK-2636771, LY-3023414, ipatasertib, afuresertib, MK-2206, MK-8156, Idelalisib, BEZ235 (dactolisib), BYL719, GDC- 0980, GDC-0941, GDC-0032 and GDC-0068.

In another embodiment is provided a compound of formula (I) in combination with one or more (e.g. 1 or 2) other therapeutic agents (e.g. anticancer agents) for use in therapy, such as in the prophylaxis or treatment of cancer.

In one embodiment the pharmaceutical composition comprises a compound of formula (I) together with a pharmaceutically acceptable carrier and optionally one or more therapeutic agent(s).

In another embodiment the invention relates to the use of a combination according to the invention in the manufacture of a pharmaceutical composition for inhibiting the growth of tumour cells.

In a further embodiment the invention relates to a product containing a compound of formula (I) and one or more anticancer agent, as a combined preparation for simultaneous, separate or sequential use in the treatment of patients suffering from cancer.

### EXAMPLES

The invention will now be illustrated, but not limited, by reference to the specific embodiments described in the following examples.

### Abbreviations

- DCM: Dichloromethane
- DMSO: Dimethylsulfoxide
- EtOAc: Ethyl acetate
- h: hour(s)
- HPLC: High-performance liquid chromatography
- KHMDS: Potassium bis(trimethylsilyl)amide
- LCMS: Liquid chromatography-mass spectrometry
- MeCN: Acetonitrile
- MeOH: Methanol
- min: minutes
- NMR: Nuclear magnetic resonance
- Pd₂(dba)₃: Tris(dibenzylideneacetone)dipalladium(0)
- PE: Petroleum ether
- rt: Room temperature or ambient temperature
- T3P: 1-Propanephosphonic anhydride solution
- THF: Tetrahydrofuran
- Xantphos: 4,5-Bis(diphenylphosphino)-9,9-dimethylxanthene

### Example 1

### (2S,3S,4S)-N-(5-Chloro-2,4-difluorophenyl)-3,4-dihydroxy-N-(methyl-d₃)-1-(6-methyl-4-(trifluoromethyl)pyridin-2-yl)-5-oxopyrrolidine-2-carboxamide (E1)

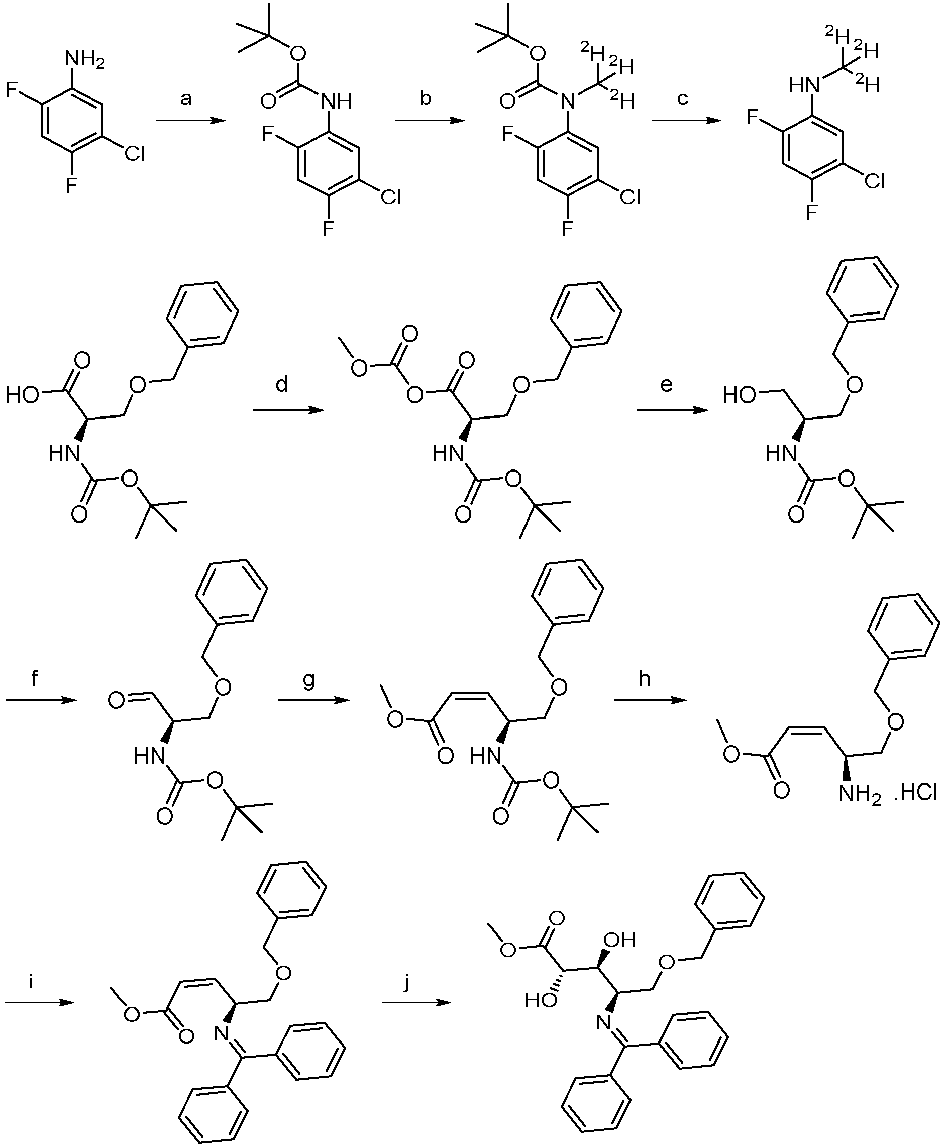

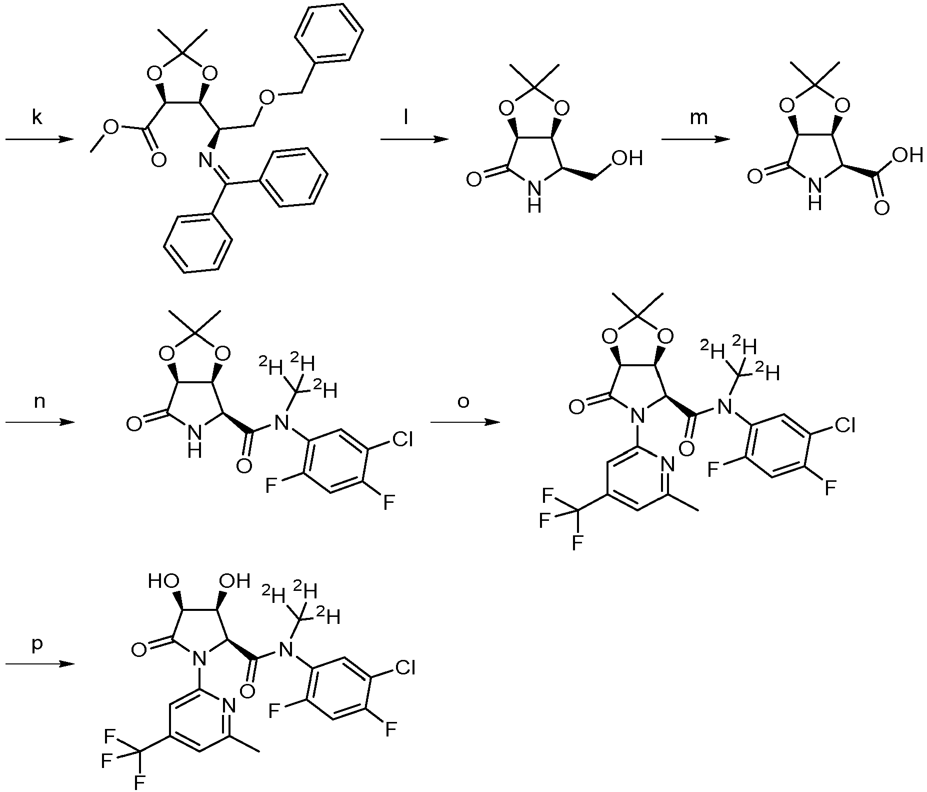

**Step a.** To a solution of 5-chloro-2,4-difluoroaniline (2.00 g, 12.2 mmol) in 1,4-dioxane (10 mL) and water (10 mL) was added di-tert-butyl dicarbonate (5.34 g, 24.5 mmol) and NaHCOs (4.11 g, 48.9 mmol). The mixture was stirred at 40°C for 12 h. On completion, the reaction mixture was concentrated under vacuum, diluted with water (20 mL) and extracted with EtOAc (20 mL x 3). The combined organic layers were washed with brine (60 mL x 2), dried over Na₂SO₄ and evaporated. The residue was purified by column chromatography (3-5% EtOAc in PE) to afford tert-butyl (5-chloro-2,4-difluorophenyl)carbamate (1.00 g, 31% yield) as a white solid.

¹H NMR (400 MHz, DMSO-d₆) δ ppm 9.21 (s, 1H), 7.82 (t, *J* = 8.0 Hz, 1H), 7.55 (dd, *J* = 9.6, 10.8 Hz, 1H), 1.45 (s, 9H).

**Step b.** To a solution of tert-butyl (5-chloro-2,4-difluorophenyl)carbamate (900 mg, 3.41 mmol) in dimethylformamide (9 mL) was added NaH (205 mg, 5.12 mmol, 60% dispersion in mineral oil) portionwise under N₂ at 0°C and the mixture was stirred at 0°C for 0.5 h. Methyl-d₃ iodide (594 mg, 4.10 mmol) was added dropwise and the mixture was stirred at 0°C for 1 h. On completion, the reaction mixture was diluted with water (10 mL) and extracted with EtOAc (10 mL x 3). The combined organic layers were washed with brine (30 mL x 3), dried and evaporated to afford tert-butyl (5-chloro-2,4-difluorophenyl)(methyl-d₃)carbamate (990 mg, crude) as a colorless oil.
m/z ES+ [M-55]⁺ 225.0

**Step c.** A solution of tert-butyl (5-chloro-2,4-difluorophenyl)(methyl-d₃)carbamate (990 mg, crude) and trifluoroacetic acid (3.08 g, 27.0 mmol) in DCM (10 mL) was stirred at rt for 0.5 h. On completion, the reaction mixture was diluted with water (15 mL) and extracted with DCM (15 mL x 3). The combined organic layers were washed with brine (40 mL x 2), dried and evaporated. The residue was purified by column chromatography (3-5% EtOAc in PE) to afford 5-chloro-2,4-difluoro-N-(methyl-d₃)aniline (490 mg, 77% yield over two steps) as a colorless oil.
m/z ES+ [M+H]⁺ 181.1

**Step d.** Into a 20L 4-necked round-bottom flask under inert atmosphere of nitrogen was added (2R)-3-(benzyloxy)-2-[(tert-butoxycarbonyl)amino]propanoic acid (CAS Number 47173-80-8; 800 g, 2.68 mol), N-methylmorpholine (298 g, 2.95 mol) and THF (8 L). The mixture was cooled to -20°C and methyl chloroformate (266 g, 2.82 mol) was added dropwise. The resulting mixture was stirred for 0.5 h at -10°C in a water/ice bath. The solids were collected by filtration to provide (2R)-3-(benzyloxy)-2-[(tert-butoxycarbonyl)amino]-1-[(methoxycarbonyl)oxy]propan-1-one, which was used in the next step without further purification.

**Step e.** Into a 20L 4-necked round-bottom flask under inert atmosphere of nitrogen was added water (8 L), which was cooled to 0°C before addition of NaBH₄ (254 g, 6.70 mol). A solution of (2R)-3-(benzyloxy)-2-[(tert-butoxycarbonyl)amino]-1-[(methoxycarbonyl)oxy]-propan-1-one (957 g, 2.68 mol) in THF (8 L) was added dropwise at 0°C. The resulting solution was stirred overnight at rt. The solids were removed by filtration. The filtrate was extracted with DCM (3 x 3 L) and the organic phase was concentrated. The crude was purified by silica gel chromatography (30% EtOAc in PE) to provide tert-butyl N-[(2S)-1-(benzyloxy)-3-hydroxypropan-2-yl]carbamate (670 g, 88% yield) as a white solid.
m/z ES+ [M+H]⁺ 282.2

**Step f.** Into a 50L 4-necked round-bottom flask under inert atmosphere of nitrogen was added a solution of (COCl)₂ (545 g, 4.29 mol) in DCM (15 L). This was followed by the addition of DMSO (670 g, 8.59 mol) dropwise with stirring at -78°C. After 0.5 h, the mixture was treated with a solution of tert-butyl N-[(2S)-1-(benzyloxy)-3-hydroxypropan-2-yl]carbamate (610 g, 2.17 mol) in DCM (3 L) dropwise with stirring at -78°C and stirred for another 0.5 h. To the mixture was added N,N-diisopropylethylamine (1664 g, 12.88 mol) dropwise with stirring at -78°C. The resulting mixture was stirred for 2 h at -78°C followed by an additional 2 h at -40°C. The reaction mixture was cooled to -70°C and quenched by the addition to 5% aqueous KHSO₄ (18 L). The resulting solution was extracted with DCM (5 L). The organic phase was washed with brine, dried over Na₂SO₄ and concentrated. The crude was used in the next step without further purification.

Note, the product was not stable in LCMS, the crude was detected by thin layer chromatography and confirmed with ¹H NMR.

**Step g.** Into a 20L 4-necked round-bottom flask under an inert atmosphere of nitrogen was added a solution of methyl 2-[bis(2,2,2-trifluoroethoxy)phosphoryl]acetate (682 g, 2.14 mol) in THF (6 L), to which 18-crown-6 (567 g, 2.14 mol) was added. This was followed by the addition of KHMDS (Hv) in THF, 2.14 L, 2.14 mol) dropwise with stirring at -78°C. To this was added a solution of tert-butyl N-[(2R)-1-(benzyloxy)-3-oxopropan-2-yl]carbamate (605 g, 2.14 mol) in THF (1.8 L) at -78°C. The resulting reaction mixture was stirred for 2 h at -78°C. The reaction was quenched by addition of 1 M HCl (12 L). The resulting solution was extracted with EtOAc (2 x 5 L), washed with water, dried over Na₂SO₄ and concentrated. The crude was purified by silica gel chromatography (30% EtOAc in PE) to provide methyl (2Z,4S)-5-(benzyloxy)-4-[(tert-butoxycarbonyl)amino]pent-2-enoate (655 g, 90% yield) as a white solid. m/z ES+ [M+H]+ 336.1; ¹H NMR (300 MHz, DMSO-d₆) δ ppm 7.36 - 7.26 (m, 5H), 6.18 - 6.12 (m, 1H), 5.90 - 5.86 (m, 1H), 5.39 - 5.32 (m, 1H), 4.55 - 4.43 (m, 2H), 3.64 (s, 3H), 3.50 - 3.39 (m, 2H), 1.47 (s, 9H).

**Step h.** Into a 5L 3-necked round-bottom flask under inert atmosphere of nitrogen was added methyl (2Z,4S)-5-(benzyloxy)-4-[(tert-butoxycarbonyl)amino]pent-2-enoate (655 g, 1.93 mol) and MeOH (3275 mL), followed by acetyl chloride (455 g, 5.80 mol) dropwise with stirring at 0°C. The resulting mixture was stirred for 12 h at rt. The resulting mixture was concentrated, the residue re-dissolved in THF and concentrated again. The crude was treated with n-hexane and solids were collected by filtration to provide methyl (2Z,4S)-4-amino-5-(benzyloxy)pent-2-enoate hydrochloride (480 g, 90% yield) as a light brown solid. m/z ES+ [M+H]⁺ 236.1; ¹H NMR (300 MHz, DMSO-d₆) δ ppm 8.58 (bs, 3H), 7.38 - 7.29 (m, 5H), 6.38 - 6.32 (m, 1H), 6.16 - 6.12 (m, 1H), 5.39 - 5.32 (m, 1H), 4.61 - 4.49 (m, 2H), 3.68 (s, 3H), 3.44 (bs, 2H).

**Step i.** Into a 5L 3-necked round-bottom flask under inert atmosphere of nitrogen was added DCM (2.40 L), methyl (2Z,4S)-4-amino-5-(benzyloxy)pent-2-enoate hydrochloride (480 g, 1.75 mol), followed by diphenylmethanimine (317 g, 1.75 mol) dropwise. The resulting mixture was stirred for 12 h at rt. The reaction mixture was concentrated to provide methyl (2Z,4S)-5-(benzyloxy)-4-[(diphenylmethylidene)amino]pent-2-enoate (780 g, crude) as light brown oil.
m/z ES+ [M+H]⁺ 400.2

**Step j.** Into a 20L 3-necked round-bottom flask under inert atmosphere of nitrogen was added methyl (2Z,4S)-5-(benzyloxy)-4-[(diphenylmethylidene)amino]pent-2-enoate (95.0 g, 1.85 mol), THF (7.80 L), water (7.80 L), N-methylmorpholine-N-oxide (543 g, 4.64 mol), followed by OsO₄ (23.6 g, 92.7 mmol) in 4 portions. The resulting mixture was stirred for 48 h at 35°C. The reaction mixture was cooled to rt. The resulting solution was extracted with EtOAc (2 x 5 L). The organic phase was washed with water (2 x 3 L). The mixture was dried over Na₂SO₄ and concentrated. The solid product was stirred in hexane and the solids were collected by filtration to provide a crude mixture of methyl (2S,3S,4R)-5-(benzyloxy)-4-[(diphenylmethylidene)amino]-2,3-dihydroxypentanoate and methyl (2R,3R,4R)-5-(benzyloxy)-4-((diphenylmethylene)amino)-2,3-dihydroxypentanoate (650 g) as a light brown solid.
m/z ES+ [M+H]⁺ 434.1

**Step k.** Into a 10L 4-necked round-bottom flask under inert atmosphere of nitrogen was added a mixture of methyl (2S,3S,4R)-5-(benzyloxy)-4-[(diphenylmethylidene)amino]-2,3-dihydroxypentanoate and methyl (2R,3R,4R)-5-(benzyloxy)-4-((diphenylmethylene)amino)-2,3-dihydroxypentanoate (650 g, 1.42 mol), toluene (6.5 L), pyridinium p-toluenesulfonate (89.5 g, 356 mmol) and 2,2-dimethoxypropane (742 g, 7.12 mol). The resulting mixture was stirred for 12 h at 100°C. The reaction mixture was concentrated. The crude product was purified by silica gel chromatography (3% EtOAc in PE) to provide methyl (4S,5S)-5-[(1R)-2-(benzyloxy)-1-[(diphenylmethylidene)amino]ethyl]-2,2-dimethyl-1,3-dioxolane-4-carboxylate 445 g (65% yield, over 3 steps) as light yellow oil.
m/z ES+ [M+H]⁺ 474.2; ¹H NMR (300 MHz, DMSO-d₆) δ ppm 7.75 - 7.16 (m, 15H), 4.65 - 4.63 (m, 1H), 4.55 - 4.50 (m, 1H), 4.31 (s, 2H), 3.74 - 3.68 (m, 1H), 3.59 - 3.57 (m, 2H), 3.26 (s, 3H), 1.54 (s, 3H), 1.33 (s, 3H).

**Step l.** Into a 10L hydrogen pressure tank reactor was added methyl (4S,5S)-5-[(1R)-2-(benzyloxy)-1-[(diphenylmethylidene)amino]ethyl]-2,2-dimethyl-1,3-dioxolane-4-carboxylate (445 g, 930 mmol), MeOH (4.45 L), 20% Pd(OH)₂/C (65 g, 93 mmol) and 10% Pd/C (99 g, 93 mmol). The resulting mixture was stirred under hydrogen atmosphere (20 atm) for 4 days at 40°C. The reaction mixture was then filtered and concentrated. The crude was stirred in hexane and then collected by filtration to provide (3aS,6R,6aS)-6-(hydroxymethyl)-2,2-dimethyl-tetrahydro-[1,3]dioxolo[4,5-c]pyrrol-4-one (153 g, 87% yield) as a white solid. m/z ES+ [M+H]⁺ 188.0; ¹H NMR (300 MHz, DMSO-d₆) δ ppm 7.89 (bs, 1H), 4.77 - 4.75 (m, 1H), 4.68 - 4.65 (m, 1H), 4.57 - 4.55 (m, 1H), 3.65 - 3.55 (m, 2H), 3.47 - 3.39 (m, 1H), 1.30 (s, 6H).

**Step m.** Into a 10L 3-necked round-bottom flask under inert atmosphere of nitrogen, was added (3aS,6R,6aS)-6-(hydroxymethyl)-2,2-dimethyl-tetrahydro-[1,3]dioxolo[4,5-c]pyrrol-4-one (153 g, 809 mmol), MeCN (1.38 L), carbon tetrachloride (1.38 L), water (2.00 L), sodium periodate (519 g, 2.43 mol) and RuCl₃ (16.8 g, 80.9 mmol). The reaction mixture was stirred for 3 h at 20-35°C. The reaction mixture was then filtered and concentrated. The crude was dissolved in MeOH, filtered and concentrated to provide (3aS,4S,6aS)-2,2-dimethyl-6-oxo-tetrahydro-[1,3]dioxolo[4,5-c]pyrrole-4-carboxylic acid (170 g, 72%) as a light brown solid. m/z ES+ [M+H]⁺ 202.2; ¹H NMR (300 MHz, DMSO-d₆) δ ppm 12.86 (s, 1H), 8.19 (s, 1H), 4.88 (t, *J* = 5.6 Hz, 1H), 4.56 (d, *J* = 5.9 Hz, 1H), 4.31 (d, *J* = 5.3 Hz, 1H), 1.27 (d, *J* = 5.4 Hz,6H); [a]_{D}= 13.2 degrees (C=0.22 g/100 mL in MeOH, T=21.2)

**Step n.** A solution of 5-chloro-2,4-difluoro-N-(methyl-d₃)aniline (2.15 g, 11.9 mmol) in N,N-dimethylacetamide (20 mL) was treated with pyridine (1.57 g, 19.8 mmol) and stirred for 15 min before addition of (3aS,4S,6aS)-2,2-dimethyl-6-oxo-tetrahydro-[1,3]dioxolo[4,5-c]pyrrole-4-carboxylic acid (2.3 g, 11.4 mmol). The reaction mixture was cooled to 0°C and treated dropwise with T3P (19 g, 30 mmol, 50% wt.% in EtOAc) with stirring at 0°C. The resulting solution was stirred at 0°C for 0.5 h and then at rt for 24 h. The reaction mixture was heated to 50°C and stirred for an additional 24 h. The reaction was quenched by addition of ice-water (80 mL), extracted with EtOAc (3 x 60 mL) and concentrated. The residue was purified by prep-HPLC (column: C18; mobile phase: A = water (5% NH₄HCO₃), B = MeCN; B%: 15-45%, 40 min) to give (3aS,4S,6aS)-N-(5-chloro-2,4-difluorophenyl)-2,2-dimethyl-N-(methyl-d₃)-6-oxotetrahydro-4H-[1,3]dioxolo[4,5-c]pyrrole-4-carboxamide.

The reaction was repeated with (3aS,4S,6aS)-2,2-dimethyl-6-oxo-tetrahydro-[1,3]dioxolo[4,5-c]pyrrole-4-carboxylic acid (3.0 g, 14.9 mmol) and the products of the two reactions combined to provide (3aS,4S,6aS)-N-(5-chloro-2,4-difluorophenyl)-2,2-dimethyl-N-(methyl-d₃)-6-oxotetrahydro-4H-[1,3]dioxolo[4,5-c]pyrrole-4-carboxamide (6.2 g, 65% yield). m/z ES+ [M+H]⁺ 364.0; ¹H NMR (400 MHz, DMSO-d₆) δ ppm 8.28 - 7.50 (m, 3H), 5.26 - 3.80 (m, 3H), 1.40 - 1.09 (m, 6H).

**Step o.** A solution of (3aS,4S,6aS)-N-(5-chloro-2,4-difluorophenyl)-2,2-dimethyl-N-(methyl-d₃)-6-oxotetrahydro-4H-[1,3]dioxolo[4,5-c]pyrrole-4-carboxamide (2.0 g, 5.5 mmol) in 1,4-dioxane was treated with 2-chloro-6-methyl-4-(trifluoromethyl)pyridine (CAS Number 22123-14-4; 1.5 g, 7.7 mmol), XantPhos (0.95 g, 1.6 mmol), Pd₂(dba)₃ (0.5 g, 0.55 mmol) and K₂CO₃ (1.52 mg, 11 mmol) and stirred at 90-95°C for 24 h. The solids were removed by filtration. The filtrate was concentrated and the residue purified by column chromatography (10% EtOAc in PE) to afford (3aS,4S,6aS)-N-(5-chloro-2,4-difluorophenyl)-2,2-dimethyl-N-(methyl-d₃)-5-(6-methyl-4-(trifluoromethyl)pyridin-2-yl)-6-oxotetrahydro-4H-[1,3]dioxolo[4,5-c]pyrrole-4-carboxamide.

The reaction was repeated with (3aS,4S,6aS)-N-(5-chloro-2,4-difluorophenyl)-2,2-dimethyl-N-(methyl-d₃)-6-oxotetrahydro-4H-[1,3]dioxolo[4,5-c]pyrrole-4-carboxamide (5.0 g, 13.7 mmol) and the products of the two reactions combined to provide (3aS,4S,6aS)-N-(5-chloro-2,4-difluorophenyl)-2,2-dimethyl-N-(methyl-d₃)-5-(6-methyl-4-(trifluoromethyl)pyridin-2-yl)-6-oxotetrahydro-4H-[1,3]dioxolo[4,5-c]pyrrole-4-carboxamide (6.5 g, 64% yield).
m/z ES+ [M+H]⁺ 523.0; ¹H NMR (300 MHz, CD₃OD) δ ppm 8.48 - 8.44 (m, 1H), 8.03 - 7.79 (m, 1H), 7.56 - 7.41 (m, 1H), 7.36 - 7.27 (m, 1H), 5.96 - 5.10 (m, 1H), 5.03 - 4.53 (m, 2H), 2.69 - 2.54 (m, 3H), 1.49 - 1.36 (m, 6H).

**Step p.** A solution of (3aS,4S,6aS)-N-(5-chloro-2,4-difluorophenyl)-2,2-dimethyl-N-(methyl-d₃)-5-(6-methyl-4-(trifluoromethyl)pyridin-2-yl)-6-oxotetrahydro-4H-[1,3]dioxolo[4,5-c]pyrrole-4-carboxamide (5.8 g, 11.1 mmol) in DCM (58 mL) was cooled to -20°C and then treated dropwise with BCl₃ (22.2 mL, 22.2 mmol, 1 M in DCM) at -20°C. The reaction mixture was warmed to rt and stirred for 2 h. The reaction was quenched by addition of saturated NaHCOs in ice-water (58 mL), extracted with DCM (3 x 29 mL) and concentrated. The residue was purified by prep-HPLC (column: C18; mobile phase: A = water, B = MeCN; B%: 20-50%, 30 min) to give the title compound.

The reaction was repeated with (3aS,4S,6aS)-N-(5-chloro-2,4-difluorophenyl)-2,2-dimethyl-N-(methyl-d₃)-5-(6-methyl-4-(trifluoromethyl)pyridin-2-yl)-6-oxotetrahydro-4H-[1,3]dioxolo[4,5-c]pyrrole-4-carboxamide (0.5 g, 0.96 mmol) and the products of the two reactions combined to provide the tilte compound (4.0 g, 69% yield).

m/z ES+ [M+H]⁺ 483.1; ¹H NMR (300 MHz, CD₃OD) δ ppm 8.40 - 8.37 (m, 1H), 8.06 - 8.01 and 7.91 - 7.86 (m x2, 1H), 7.53 - 7.43 (m, 1H), 7.30 - 7.24 (m, 1H), 5.82, 5.19 and 5.03 (d x3, J = 5.4 Hz, 1H), 4.45 and 4.28 (dd, J = 6.8 Hz, 1H), 4.25 - 4.08 (m, 1H), 2.67 - 2.53 (m, 3H). The NMR spectra for Example 1 is presented in Figure 1.

### BIOLOGICAL DATA

### PolΘ Full Length Enzyme Potency Assay

PicoGreen assay was used to measure the ability of compounds to inhibit the activity of PolΘ in vitro. N-His, C-term FLAG tagged PolΘ protein (amino acids 2-2590) expressed in baculovirus was purified and stored at -80°C in aliquots. Assay measurements were performed with 1X buffer comprising 25 mM Tris HCl pH 7.5, 12.5 mM NaCl, 0.5 mM MgCl₂, 5% glycerol, 0.01% Triton X-100, 0.01% BGG and 1 mM DTT. Test compounds were prepared by dilution in 100% DMSO to give the correct dose range for 12 point concentration response and appropriate volume (60 nL) dispensed into 384 well micro assay plates (Perkin Elmer low volume black ProxiPlates product code 6008269) using a Labcyte Echo 550 acoustic dispenser. DMSO concentration was maintained at 1% by back filling with DMSO solution. 3 µL purified recombinant PolΘ and primer (5' - GCG GCT GTC ATA AG - 3' (SEQ ID NO: 1)): template (5' - GCT ACA TTG ACA ATG GCA TCA AAT CTC AGA TTG CGT CTT ATG ACA GCC GCG - 3' (SEQ ID NO: 2)) duplex (1:1.1) was diluted in assay buffer to a 2X working concentration (4 nM PolΘ and 100 nM PTD). This was dispensed into each well of the compound plate using a VIAFLO 16 channel manual pipette (Integra) and preincubated at rt for 30 min. 3 µL of 2X working solution of dNTPs (40 µM) (dATP, dCTP, dGTP, dTTP; Sigma D6500, D4635, D4010, T0251) diluted in assay buffer was then added and the reaction incubated for 60 min at rt. The reaction was stopped by addition of 10 mM EDTA, 25 mM Tris pH 7.5 and 1:200 dilution of PicoGreen dye (Invitrogen P7581). After 90 minutes at rt in the dark, fluorescence was read on a BMG Pherastar FS plate reader using 485/520nm module and raw data analysed using IDBS Activity Base to generate IC₅₀ values.

The compound of **Example 1** was tested in the above mentioned enzyme potency assay and the results are shown in the following table:

| **Example Number** | **PolΘ IC₅₀ (nM)** | **PolΘ pIC₅₀** | **n** | **PolΘ pIC₅₀ (SD)** |
|---|---|---|---|---|
| 1 | 12.2 | 7.92 | 15 | 0.157 |

## Claims

1. A deuterated derivative of a compound of formula (I): 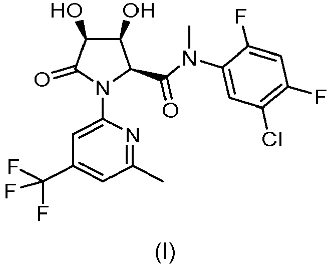 or a tautomeric or a stereochemically isomeric form thereof, or a pharmaceutically acceptable solvate thereof.

2. The compound according to claim 1, wherein the deuterated derivative comprises deuteration of one or more (such as all 3) hydrogen atoms of the N-methyl group.

3. The compound according to claim 2, wherein the deuterated derivative is a compound of formula (II): 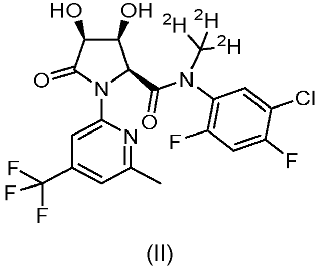 or a tautomeric or a stereochemically isomeric form, or a pharmaceutically acceptable solvate thereof.

4. The compound according to claim 3, which is the free base of a compound of formula (II) and is (2S,3S,4S)-N-(5-chloro-2,4-difluorophenyl)-3,4-dihydroxy-N-(methyl-d₃)-1-(6-methyl-4-(trifluoromethyl)pyridin-2-yl)-5-oxopyrrolidine-2-carboxamide (E1).

5. A pharmaceutical composition comprising a compound of formula (I) according to any of claims 1 to 4.

6. A pharmaceutical composition comprising a compound of formula (I) according to any of claims 1 to 4, in combination with one or more therapeutic agents.

7. A compound according to any of claims 1 to 4 for use in therapy.

8. A compound according to any of claims 1 to 4 for use in the prophylaxis or treatment of cancer.

9. A process for preparing the deuterated derivative of a compound of formula (I) according to claim 1 which comprises:
(a) reacting a deuterated derivative of a compound of formula (III): 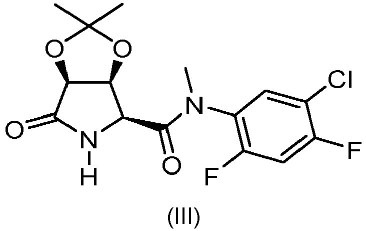 with a compound of formula (IV): 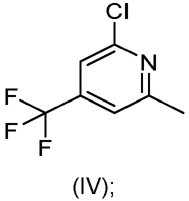
(b) deprotection of a protected derivative of a compound of formula (I); and
(c) interconversion of a compound of formula (I) or protected derivative thereof to a further compound of formula (I) or protected derivative thereof.

10. The process as defined in claim 9, wherein the compound of formula (III) is a compound of formula (III)^{a}:

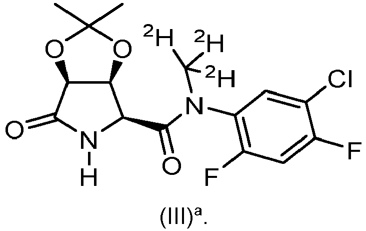

## Patentansprüche

1. Deuteriertes Derivat einer Verbindung der Formel (I): 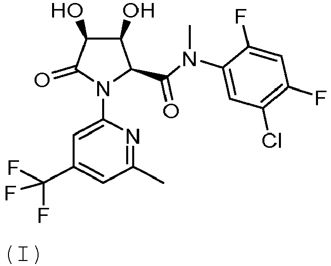 oder tautomere oder stereochemisch isomere Form davon oder pharmazeutisch verträgliches Solvat davon.

2. Verbindung nach Anspruch 1, wobei das deuterierte Derivat eine Deuterierung von einem oder mehreren (wie etwa allen 3) Wasserstoffatomen der N-Methylgruppe umfasst.

3. Verbindung nach Anspruch 2, wobei das deuterierte Derivat eine Verbindung der Formel (II) ist: 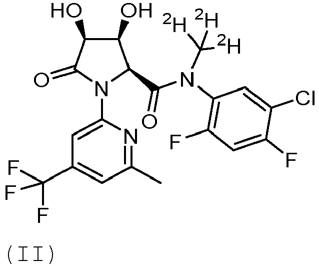 oder tautomere oder stereochemisch isomere Form oder pharmazeutisch verträgliches Solvat davon.

4. Verbindung nach Anspruch 3, die die freie Base einer Verbindung der Formel (II) ist und (2S,3S,4S)-N-(5-Chlor-2,4-difluorphenyl)-3,4-dihydroxy-N-(methyl-d₃)-1-(6-methyl-4-(trifluormethyl)pyridin-2-yl)-5-oxopyrrolidin-2-carboxamid (E1) ist.

5. Pharmazeutische Zusammensetzung, die eine Verbindung der Formel (I) nach einem der Ansprüche 1 bis 4 umfasst.

6. Pharmazeutische Zusammensetzung, die eine Verbindung der Formel (I) nach einem der Ansprüche 1 bis 4 in Kombination mit einem oder mehreren therapeutischen Mitteln umfasst.

7. Verbindung nach einem der Ansprüche 1 bis 4 zur Verwendung bei der Therapie.

8. Verbindung nach einem der Ansprüche 1 bis 4 zur Verwendung bei der Prophylaxe oder Behandlung von Krebs.

9. Verfahren zur Herstellung des deuterierten Derivats einer Verbindung der Formel (I) nach Anspruch 1, das Folgendes umfasst:
(a) Umsetzen eines deuterierten Derivats einer Verbindung der Formel (III): 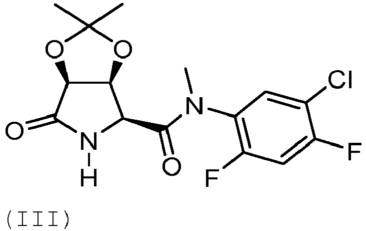 mit einer Verbindung der Formel (IV): 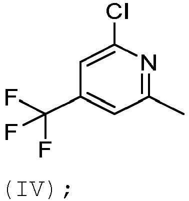
(b) Entschützung eines geschützten Derivats einer Verbindung der Formel (I); und
(c) Umwandlung einer Verbindung der Formel (I) oder eines geschützten Derivats davon in eine weitere Verbindung der Formel (I) oder ein geschütztes Derivat davon.

10. Verfahren nach Anspruch 9, wobei die Verbindung der Formel (III) eine Verbindung der Formel (III)^{a} ist:

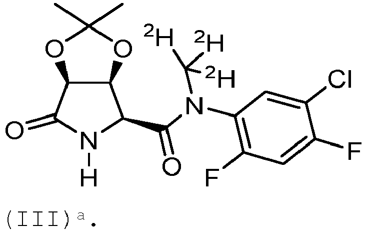

## Revendications

1. Dérivé deutéré d'un composé de formule (I) : 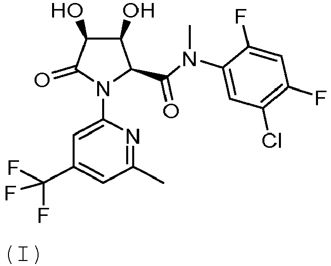 ou une forme tautomère ou stéréochimiquement isomère de celui-ci, ou un solvate pharmaceutiquement acceptable de celui-ci.

2. Composé selon la revendication 1, dans lequel le dérivé deutéré comprend la deutération d'un ou plusieurs (par exemple tous les 3) atomes d'hydrogène du groupe N-méthyle.

3. Composé selon la revendication 2, dans lequel le dérivé deutéré est un composé de formule (II) : 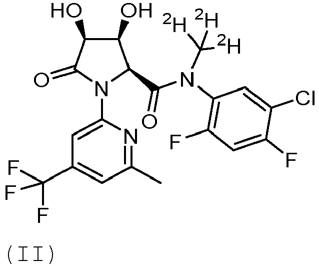 ou une forme tautomère ou stéréochimiquement isomère, ou un solvate pharmaceutiquement acceptable de celui-ci.

4. Composé selon la revendication 3, qui est la base libre d'un composé de formule (II) et est le (2S,3S,4S)-N-(5-chloro-2,4-difluorophényl)-3,4- dihydroxy-N-(méthyl-d₃)-1-(6-méthyl-4-(trifluorométhyl)pyridin-2-yl)-5-oxopyrrolidine-2-carboxamide (E1) .

5. Composition pharmaceutique comprenant un composé de formule (I) selon l'une quelconque des revendications 1 à 4.

6. Composition pharmaceutique comprenant un composé de formule (I) selon l'une quelconque des revendications 1 à 4, en combinaison avec un ou plusieurs agents thérapeutiques.

7. Composé selon l'une quelconque des revendications 1 à 4 destiné à être utilisé en thérapie.

8. Composé selon l'une quelconque des revendications 1 à 4 destiné à être utilisé dans la prophylaxie ou le traitement du cancer.

9. Procédé de préparation du dérivé deutéré d'un composé de formule (I) selon la revendication 1 qui comprend :
(a) la réaction d'un dérivé deutéré d'un composé de formule (III) : 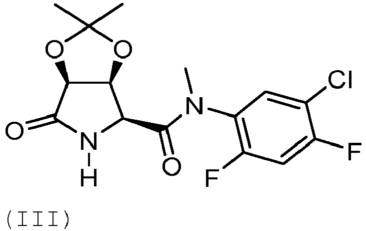 avec un composé de formule (IV) : 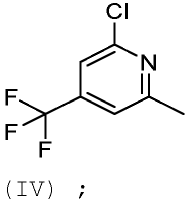
(b) la déprotection d'un dérivé protégé d'un composé de formule (I) ; et
(c) l'interconversion d'un composé de formule (I) ou d'un dérivé protégé de celui-ci en un autre composé de formule (I) ou en un dérivé protégé de celui-ci.

10. Procédé selon la revendication 9, dans lequel le composé de formule (III) est un composé de formule (III)^{a} :
